(19) Europäisches Patentamt
European Patent Office
Office européen des brevets

(11) EP 3 298 138 B1

(12) # EUROPEAN PATENT SPECIFICATION

(45) Date of publication and mention of the grant of the patent:
**10.08.2022 Bulletin 2022/32**

(21) Application number: **16736945.3**

(22) Date of filing: **18.05.2016**

(51) International Patent Classification (IPC):
***C12G 1/022*** *(2006.01)* ***C12N 1/38*** *(2006.01)*
***C12N 9/96*** *(2006.01)* ***C12C 5/00*** *(2006.01)*
***C12N 9/88*** *(2006.01)*

(52) Cooperative Patent Classification (CPC):
**C12N 9/96; C12C 5/00; C12C 5/004; C12G 1/0203; C12N 1/38; C12N 9/88; C12Y 401/01005;**
C12G 2200/15

(86) International application number:
**PCT/US2016/033028**

(87) International publication number:
**WO 2016/191169 (01.12.2016 Gazette 2016/48)**

(54) **ACETOLACTATE DECARBOXYLASE**

ACETOLACTAT-DECARBOXYLASE

ACETOLACTATE DECARBOXYLASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.05.2015 US 201562165671 P**
**26.05.2015 US 201562166610 P**
**29.05.2015 US 201562168406 P**

(43) Date of publication of application:
**28.03.2018 Bulletin 2018/13**

(73) Proprietor: **DuPont Nutrition Biosciences ApS**
**1411 Copenhagen K (DK)**

(72) Inventors:
- **CRAMER, Jacob Flyvholm**
  **8220 Brabrand (DK)**
- **JENSEN, Lene Bojsen**
  **8270 Højbjerg (DK)**
- **KELLETT-SMITH, Anja Hemmingsen**
  **Palo Alto, CA 94304 (US)**
- **BLADT, Tove**
  **8660 Skanderborg (DK)**
- **LEE, Sang-Kyu**
  **Mountain View, California 94041 (US)**

(74) Representative: **D Young & Co LLP**
**120 Holborn**
**London EC1N 2DY (GB)**

(56) References cited:
**EP-A2- 0 128 714**
**WO-A1-2013/092840**
**WO-A1-2014/092562**
**KR-B1- 100 870 561**
**US-B2- 8 313 926**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 298 138 B1

## Description

## BACKGROUND

Diacetyl is sometimes an unwanted by-product of fermentation processes of carbohydrate containing substances, e.g. wort or grape juice. Formation of diacetyl is most disadvantageous because of its strong and unpleasant smell and in case of beer even small amounts of diacetyl of about 0.10 to 0.15 mg/liter has a negative effect on the flavor and taste of the beer. During the maturation of beer, diacetyl is converted into acetoin by reductases in the yeast cells. Acetoin is with respect to taste and flavor acceptable in beer in much higher concentrations than diacetyl.

Acetolactate decarboxylase (ALDC) can also be used as an enzyme to prevent the formation of diacetyl. $\alpha$-acetolactate can be converted into acetoin by adding an ALDC enzyme during fermentation. However, ALDC can be unstable at fermenting conditions, especially those of fermenting worts with low malt content.

The purpose of the present invention is to provide ALDC enzymes having a better stability and/or activity, and, optionally, the yield of ALDC enzymes which can be recovered from microorganisms is improved.

## SUMMARY OF THE INVENTION

The present disclosure provides compositions and processes for ALDC enzymes. The present invention is defined in the appended claims. Information extending beyond the scope of the claims is provided for information only. The present invention provides a method for increasing the activity and/or stability of an ALDC enzyme of EC 4.1.1.5 by adding zinc, in a concentration of 1 $\mu$M to 5 mM or at a molar ratio of zinc to ALDC enzyme in the composition of higher than 1, to a fermentation media and/or maturation media comprising an ALDC producing host cell during a beer and/or wine and/or cider and/or perry and/or sake fermentation. The present invention further provides a beer and/or wine and/or cider and/or perry and/or sake fermentation media and/or maturation media comprising a composition comprising an ALDC enzyme of EC 4.1.1.5 and zinc wherein said composition comprises zinc at a concentration of 1 $\mu$M to 200 mM.

## BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:

**Figure 1** shows a plasmid map of alrA(CB)RIHI-aldB for expression of Acetolactate Decarboxylase, aldB

**Figure 2** shows a graph depicting the activity of ALDC relative to 0 mM zinc vs concentration of zinc in mM.

**Figure 3** shows SDS-PAGE with aldB samples containing varying concentration of $ZnSO_4$. Gel A - Lane 1) Molecular weight marker; Lane 2-7) BSA standard; Lane 8-9) AldB with 0 mM $ZnSO_4$; Lane 10-11) AldB with 0.25 mM $ZnSO_4$; Lane 12-13) AldB with 0.5 mM $ZnSO_4$; Lane 14-15) AldB with 1.0 mM $ZnSO_4$; Lane 16-17) AldB with 2 mM $ZnSO_4$; Lane 18-19) AldB with 5 mM $ZnSO_4$; Lane 20-21) AldB with 7.5 mM $ZnSO_4$; Lane 22-23) AldB with 10 mM $ZnSO_4$ and Lane 24-25) AldB with 20 mM $ZnSO_4$. Gel B - Lane 1) Molecular weight marker; Lane 2-7) BSA standard; Lane 8-11) AldB with 0 mM $ZnSO_4$; Lane 12-15) AldB with 20 mM $ZnSO_4$; Lane 16-17) AldB with 40 mM $ZnSO_4$; Lane 18-19) AldB with 60 mM $ZnSO_4$; Lane 20-21) AldB with 80 mM $ZnSO_4$; Lane 22-23) AldB with 100 mM $ZnSO_4$ and Lane 24-25) AldB with 120 mM $ZnSO_4$.

**Figure 4** shows SDS-PAGE with purification of aldB produced in B. subtilis. Lane 1) crude aldB ferment; 2-3) purified aldB from Sourcel5Q. Molecular weight marker is shown to the left and in-between lane 1 and 2.

**Figure 5** shows development of vicinal diketones (VDK) during malt-based fermentations in the presence or absence of 0.03 U/mL wort aldB enzyme variants with different specific activity: A) 919 U/mg, B) 1103 U/mg and C) 1552 U/mg aldB. The VDK development (sum of diacetyl and 2,3 pentanedione) was followed during the 7 days of fermentation at 14°C. The average VDK values are calculated from duplicate samples and labels are shown as insert in figure.

**Figure 6** shows VDK development in presence aldB enzyme (0.04 U/mL wort) during malt-based fermentations with different levels of $Zn^{2+}$ in the wort (see labels). The VDK development (sum of diacetyl and 2,3-pentanedione) was followed during the 7 days of fermentation at 14°C. The average VDK values are calculated from duplicate samples and labels are shown as insert in figure.

## DETAILED DESCRIPTION OF THE INVENTION

The present disclosure provides methods, compositions, apparatuses and kits comprising ALDC enzymes having a better stability and/or activity, and, optionally, the yield of ALDC enzymes which can be recovered from microorganisms is improved. In some embodiments, the present disclosure provides methods, apparatuses, compositions and kits for the use of metal ions to increase stability and/or activity, and, optionally, which further can be used to recover the enzymes (e.g. ALDC enzymes) from microorganisms in improved yields.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

It must be noted that as used herein and in the appended claims, the singular forms "a", "an", and "the" include plural referents unless the context clearly dictates otherwise. Thus, for example, reference to "a protease" includes a plurality of such enzymes and reference to "the feed" includes reference to one or more feeds and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The invention is defined by the claims. Information extending beyond the scope of the claims is provided for information only.

## ALDC

In some aspects the description provides ALDC enzymes having a better stability and/or activity, and, optionally, the yield of ALDC enzymes which can be recovered from microorganisms is improved. The terms "better stability" and "increased stability" as used herein refer to an ALDC enzyme whose ALDC activity is maintained for a longer period of time when in the presence of a metal ion (such as $Zn^{2+}$) when compared to the ALDC activity of the enzyme in the absence of the metal ion. The terms "better activity" and "increased activity" as used herein refer to an ALDC enzyme having an increased ALDC activity when in the presence of a metal ion (such as $Zn^{2+}$) when compared to the ALDC activity of the enzyme in the absence of the metal ion. The term "improved" in connection to the yield of ALDC enzyme refers to an increase in the ALDC activity which is produced when a host microorganism is in the presence of a metal ion (such as $Zn^{2+}$) compared to the ALDC activity produced when the host microorganism is in the absence of the metal ion. Without wishing to be bound by theory, the metal ion (such as $Zn^{2+}$) can be added during and/or after the culture process (e.g. ALDC production) in order to increase stability and/or increase activity and/or to increase yield of ALDC enzymes. The terms "host cell", "host microorganism", "strain" and "microorganism" may be used interchangeably herein.

Acetolactate decarboxylase (ALDC) is an enzyme that belongs to the family of carboxy lyases, which are responsible for cleaving carbon-carbon bonds. Acetolactate decarboxylase catalyzes the conversion of 2-acetolactate (also known as 2-hydroxy-2-methyl-3-oxobutanoate) to 2-acetoin and releases $CO_2$. The terms "ALDC" and "ALDC enzyme" may be used interchangeably herein.

Acetolactate decarboxylase enzymes catalyze the enzymatic reaction belonging to the classification EC 4.1.1.5 (acetolactate decarboxylase activity) and gene ontology (GO) term ID of GO: 0047605. The GO term ID specifies that any protein characterized as having this associated GO term encodes an enzyme with catalytic acetolactate decarboxylase activity.

Various acetolactate decarboxylase genes *(*such as *alsD* or *aldB*), which encode acetolactate decarboxylase enzymes, are known in the art. The *alsD* gene, which encodes ALDC enzyme, may be derived or derivable from *Bacillus subtilis.* The *aldB* gene, which encodes ALDC enzyme, may be derived or derivable from *Bacillus brevis.* The *alsD* gene, which encodes ALDC enzyme, may be derived or derivable from *Bacillus licheniformis*. UNIPROT accession number Q65E52.1 is an example of an ALDC enzyme. UNIPROT accession number Q65E52.1 is an example of an ALDC enzyme derived or derivable from *Bacillus licheniformis.* Examples of acetolactate decarboxylase genes include but are not limited to gi|375143627|ref|YP_005006068.1| acetolactate decarboxylase *[Niastella koreensis* OR20-10]; gi|361057673|gb|AEV96664.1| acetolactate decarboxylase *[Niastella koreensis* OR20-10]; gi|218763415|gb|ACL05881.1| acetolactate decarboxylase *[Desulfatibacillum alkenivorans* AK-01];

gi|220909520|ref|YP_002484831.1| acetolactate decarboxylase *[Cyanothece sp.* PCC 7425]; gi|218782031|ref|YP_002433349.1| acetolactate decarboxylase *[Desulfatibacillum alkenivorans* AK-01]; gi|213693090|ref|YP_002323676.1| acetolactate decarboxylase *[Bifidobacterium longum subsp. infantis* ATCC 15697 = JCM 1222]; gi|189500297|ref|YP_001959767.1| acetolactate decarboxylase *[Chlorobium phaeobacteroides* BS1]; gi|189423787|ref|YP_001950964.1| acetolactate decarboxylase *[Geobacter lovleyi* SZ]; gi|172058271|ref|YP_001814731.1| acetolactate decarboxylase *[Exiguobacterium sibiricum* 255-15]; gi|163938775|ref|YP_001643659.1| acetolactate decarboxylase *[Bacillus weihenstephanensis* KBAB4]; gi|158522304|ref|YP 001530174.1| acetolactate decarboxylase *[Desulfococcus oleovorans* Hxd3]; gi|57371670|ref|YP_001479659.1| acetolactate decarboxylase *[Serratia proteamaculans* 568]; gi|150395111|ref|YP_001317786.1| acetolactate decarboxylase [Staphylococcus aureus subsp. aureus JHl]; gi|150394715|ref|YP_001317390.1| acetolactate decarboxylase *[Staphylococcus aureus subsp. aureus* JH1]; gi|146311679|ref|YP_001176753.1| acetolactate decarboxylase *[Enterobacter sp.* 638]; gi|109900061|ref|YP_663316.1| acetolactate decarboxylase *[Pseudoalteromonas atlantica* T6c]; gi|219866131|gb|ACL46470.1| acetolactate decarboxylase *[Cyanothece sp.* PCC 7425]; gi|213524551|gb|ACJ53298.1| acetolactate decarboxylase *[Bifidobacterium longum* subsp. *infantis* ATCC 15697 = JCM 1222]; gi|189420046|gb|ACD94444.1| acetolactate decarboxylase *[Geobacter lovleyi* SZ]; gi|158511130|gb|ABW68097.1| acetolactate decarboxylase *[Desulfococcus oleovorans* Hxd3]; gi|157323434|gb|ABV42531.1| acetolactate decarboxylase *[Serratia proteamaculans* 568]; gi|145318555|gb|ABP60702.1| acetolactate decarboxylase *[Enterobacter sp.* 638*]*; gi|149947563|gb|ABR53499.1| acetolactate decarboxylase *[Staphylococcus aureus subsp.aureus* JH1]; gi|149947167|gb|ABR53103.1| acetolactate decarboxylase *[Staphylococcusaureus subsp. aureus* JH1]; gi|163860972|gb|ABY42031.1| Acetolactate decarboxylase *[Bacillus weihenstephanensis* KBAB4]; gi|109702342|gb|ABG42262.1| Acetolactate decarboxylase *[Pseudoalteromonas atlantica* T6c]; gi|189495738|gb|ACE04286.1| acetolactate decarboxylase *[Chlorobium phaeobacteroides* BS1]; gi|171990792|gb|ACB61714.1| acetolactate decarboxylase *[Exiguobacterium sibiricum* 255-15]; gi|223932563|ref|ZP_03624564.1 acetolactate decarboxylase *[Streptococcus suis* 89/1591]; gi|194467531|ref|ZP_03073518.1| acetolactate decarboxylase *[Lactobacillus reuteri* 100-23]; gi|223898834|gb|EEF65194.1| acetolactate decarboxylase *[Streptococcus suis* 89/1591]; gi|194454567|gb|EDX43464.1| acetolactate decarboxylase *[Lactobacillus reuteri* 100-23]; gi|384267135|ref|YP_005422842.1| acetolactate decarboxylase *[Bacillus amyloliquefaciens subsp. plantarum* YAU B9601-Y2]; gi[375364037|rel| YP_005132076.1| acetolactate decarboxylase *[Bacillus amyloliquefaciens subsp. plantarum* CAU B946]; gi|34079323|ref|YP_004758694.1| acetolactate decarboxylase *[Corynebacterium variabile* DSM 44702]; gi|336325119|ref|YP_004605085.1| acetolactate decarboxylase *[Corynebacterium resistens* DSM 45100]; gi|148269032|ref|YP_001247975.1| acetolactate decarboxylase *[Staphylococcus aureus subsp. aureus* JH9]; gi|148268650|ref|YP_001247593.1| acetolactate decarboxylase *[Staphylococcus aureus subsp. aureus* JH9]; gi|1485433721|ref|YP_001270742.1| acetolactate decarboxylase *[Lactobacillus reuteri* DSM 20016]; gi|380500488|emb|CCG51526.1| acetolactate decarboxylase *[Bacillus amyloliquefaciens subsp. plantarum* YAU B9601-Y2]; gi|371570031|emb|CCF06881.1| acetolactate decarboxylase *[Bacillus amyloliquefaciens subsp. plantarum* CAU B946]; gi|340533141|gb|AEK35621.1| acetolactate decarboxylase *[Corynebacterium variabile* DSM 44702]; gi|336101101|gb|AE108921.1| acetolactate decarboxylase *[Corynebacterium resistens* DSM 45100]; gi|148530406|gb|ABQ82405.1| acetolactate decarboxylase *[Lactobacillus reuteri* DSM 20016]; gi|147742101|gb|ABQ50399.1| acetolactate decarboxylase *[Staphylococcus aureus subsp. aureus* JH9]; gi|147741719|gb|ABQ50017.1| acetolactate decarboxylase *[Staphylococcus aureus subsp. aureus* JH9]; gi|392529510|ref|ZP_10276647.1| acetolactate decarboxylase *[Carnobacterium maltaromaticum* ATCC 35586]; gi|366054074|ref|ZP_09451796.1| acetolactate decarboxylase *[Lactobacillus suebicus* KCTC 3549]; gi|339624147|ref|ZP_08659936.1| acetolactate decarboxylase *[Fructobacillus jructosus* KCTC 3544]; and gi|336393727|ref|ZP_08575126.1| acetolactate decarboxylase *[Lactobacillus coryniformis subsp. torquens* KCTC 3535]. UNIPROT Accession No. P23616.1 (Diderichsen et al., J Bacteriol. (1990) 172(8): 4315) is an example of an ALDC enzyme. UNIPROT accession number P23616.1 is an example of an ALDC enzyme derived or derivable from *Bacillus brevis.*

In some embodiments, the invention relates to ALDC enzymes from *Lactobacillus casei* (Godtfredsen 1984), *Brevibacterium acetylicum* (Oshiro, 1989), *Lactococcus lactis* (Vincent Phalip 1994), *Leuconostoc lactis* (O sulivan, 2001), *Enterobacter aerogenes* (Blomquist, 1993), *Bacillus subtilis* (Renna, 1993), *Bacillus brevis* (Svendsen, 1989) and *Lactococcus lactis DX* (Yuxing, 2014). In some embodiments, the ALDC enzyme is from *Lactobacillus casei, Brevibacterium acetylicum, Lactococcus lactis, Leuconostoc lactis, Enterobacter aerogenes, Bacillus subtilis, Bacillus brevis, Lactococcus lactis DX,* or *Bacillus licheniformis.* As used herein, the term "ALDC enzyme is from" refers to the ALDC enzyme being derived or derivable from.

It is to be understood that any suitable ALDC enzymes, i.e. ALDC produced from any microorganism which activity is dependent on metal ions, can be used according to the invention. In some embodiments, the ALDC used in the methods and compositions described herein is an ALDC from *Bacillus brevis* or *Bacillus licheniformis.*

The ALDC activity of the enzyme composition according to the description is measured by the ALDC assays as described herein or any suitable assay known in the art. The standard assay is carried out at pH 6.0, and it can be performed at different pH values and temperatures for the additional characterization and specification of enzymes.

One unit of ALDC activity is defined as the amount of enzyme which produces 1 μmole acetoin per minute under the conditions of the assay (e.g., pH 6.0 (or as specified) and 30 °C).

In some embodiments, the ALDC is an ALDC derivative. In some embodiments, the ALDC derivative is characterized by the fact that ALDC in an aqueous medium is treated with or has been treated with glutaraldehyde. In some embodiments, the ALDC is treated with or has been treated with glutaraldehyde in a concentration corresponding to between 0.1 and 5 g of glutaraldehyde per e of pure ALDC protein, preferably corresponding to between 0.25 and 2 g of glutaraldehyde per g of pure ALDC protein.

In some embodiments, the ALDC enzyme comprises an amino acid sequence having at least 70%, 75%, 80%, 85%, 90%, 95%, 97%, 98%, 99% or 100% identity with SEQ ID NO: 2, SEQ ID NO: 3, SEQ ID NO: 5, SEQ ID NO: 7, or SEQ ID NO: 8 or any functional fragment thereof. In some embodiments, the ALDC enzyme is encoded by a nucleic acid sequence having at least 70%, 75%, 80%, 85%, 90%, 95%, 97%, 98%, 99% or 100% identity with SEQ ID NO: 1, SEQ ID NO: 4, or SEQ ID NO: 6 or any functional fragment thereof.

In some embodiments, the enzyme has a temperature optimum in the range of 5-80 °C, such as in the range of 5-40°C or 15-80°C, such as in the range 20-80 °C, such as in the range 5-15°C, 10-40°C, 10-50°C, 15-20°C, 45-65 °C, 50-65 °C, 55-65 °C or 60-80°C. In some embodiments, the enzyme has a temperature optimum of about 60°C.

In some embodiments, the enzyme has a total number of amino acids of less than 350, such as less than 340, such as less than 330, such as less than 320, such as less than 310, such as less than 300 amino acids, such as in the range of 200 to 350, such as in the range of 220 to 345 amino acids.

In some embodiments, the amino acid sequence of the enzyme has at least one, two, three, four, five, six, seven, eight, nine or ten amino acid substitutions as compared to any one amino acid sequence selected from SEQ ID NO: 2, SEQ ID NO: 3, SEQ ID NO: 5, SEQ ID NO: 7, SEQ ID NO: 8, or any functional fragment thereof.

In some embodiments, the amino acid sequence of the enzyme has a maximum of one, two, three, four, five, six, seven, eight, nine or ten amino acid substitutions compared to any one amino acid sequence selected from SEQ ID NO: 2, SEQ ID NO: 3, SEQ ID NO: 5, SEQ ID NO: 7, SEQ ID NO: 8, or any functional fragment thereof.

In some embodiments, the enzyme comprises the amino acid sequence identified by any one of SEQ ID NO: 2, SEQ ID NO: 3, SEQ ID NO: 5, SEQ ID NO: 7, or SEQ ID NO: 8, or any functional fragment thereof.

In some embodiments the media and methods according to the invention comprise any one or more further enzyme. In some embodiments the one or more further enzyme is selected from list consisting of acetolactate reductoisomerases, acetolactate isomerases, amylase, glucoamylase, hemicellulase, cellulase, glucanase, pullulanase, isoamylase, endo-glucanase and related beta-glucan hydrolytic accessory enzymes, xylanase, xylanase accessory enzymes (for example, arabinofuranosidase, ferulic acid esterase, xylan acetyl esterase) and protease.

In some embodiments the media and methods according to the invention comprise an enzyme exhibiting ALDC activity, wherein the activity of said ALDC enzyme is in the range of 950 to 2500 Units per mg of protein. In some embodiments the media and methods according to the invention comprise an enzyme exhibiting ALDC activity, wherein the activity of said ALDC enzyme is in the range of 1000 to 2500 Units per mg of protein. In some embodiments the media and methods according to the invention comprise an enzyme exhibiting ALDC activity, wherein the activity of said ALDC enzyme is in the range of 1500 to 2500 Units per mg of protein. In some embodiments, the enzyme exhibiting ALDC activity is an enzyme comprising an amino acid sequence having at least 80% identity with any one selected from SEQ ID NO: 2, SEQ ID NO: 3, SEQ ID NO: 5, SEQ ID NO: 7, SEQ ID NO: 8, or any functional fragment thereof. In some embodiments, the enzyme exhibiting ALDC activity is encoded by a nucleic acid sequence having at least 80% identity with SEQ ID NO: 1, SEQ ID NO: 4, SEQ ID NO: 6 or any functional fragment thereof.

### *Metal Ions*

In one aspect, the invention provides methods comprising ALDC enzymes having a better stability and/or activity.

Surprisingly, it has been found by the present inventors that treatment of ALDC compositions with certain metal ions at certain concentrations provides ALDC enzymes having a better stability and/or activity, and, optionally, the yield of ALDC activity which can be recovered from microorganisms is improved.

In some embodiments, the atomic radius for the metal ion is about 140 pm to about 255 pm. In some embodiments, the atomic radius for the metal ion is about 140 pm to about 165 pm. In some embodiments, the atomic radius for the metal ion is about 140 pm to about 150 pm. In some embodiments, the atomic radius for the metal ion is about 142 pm to about 146 pm.

For information only, examples of metal ions not in accordance with the claimed invention include $Mg^{2+}$, $Mn^{2+}$, $Co^{2+}$, $Cu^{2+}$, $Ba^{2+}$, $Ca^{2+}$ and $Fe^{2+}$ and combinations thereof. In the media and methods of the invention, the metal ion is $Zn^{2+}$. The term "zinc" as used herein may be interchangeable with the term "$Zn^{2+}$". The term "metal" as used herein may be interchangeable with the term "metal ion". The term "metal" as used herein may refer to compounds which comprise the metal selected from the group consisting of zinc, magnesium, manganese, cobalt, copper, barium, calcium and iron; compounds which comprise these metals are a source of the respective ions. The term "zinc" as used herein refers to compounds which comprise zinc, such compounds are a source of $Zn^{2+}$ ions. Zinc sulfate ($ZnSO_4$) is example of zinc as referred to herein and is an example of a source of $Zn^{2+}$ ions. Magnesium sulfate ($MgSO_4$) is an example of magnesium as referred to herein and is an example of a source of $Mg^{2+}$ ions. Manganese(II) sulfate ($MnSO_4$) is an example of manganese as referred to herein and is an example of a source of $Mn^{2+}$ ions. Cobalt(II)chloride ($CoCl_2$) is an example of cobalt as referred to herein and is an example of a source of $Co^{2+}$ ions. Copper(II) sulphate ($CuSO_4$) is an example of copper as referred to herein and is an example of a source of $Cu^{2+}$ ions. Barium sulfate ($BaSO_4$) is an example of barium as referred to herein and is an example of a source of $Ba^{2+}$ ions. Calcium sulfate ($CaSO_4$) is an example of calcium as referred to herein and is example of a source of $Ca^{2+}$ ions. Iron(II) sulfate ($FeSO_4$) is an example of iron as referred to herein and is example of a source of $Fe^{2+}$ ions.

The present inventors have found that metal ions such as $Zn^{2+}$, $Mn^{2+}$, $Co^{2+}$, $Cu^{2+}$, and $Fe^{2+}$ increase the stability of ALDC enzyme in different formulations (see Examples), and also improve the recovery yields from microorganisms when the metal ions are used during the production of the enzyme in the cultivation media. Thus, in some embodiments, the present invention provides methods that increase the recovery yields, stability and/or activity of ALDC enzymes that can be then used, e.g., to produce fermented products such as in brewing.

In some embodiments, the ALDC enzyme has an specific activity of at least about 900 units per mg of protein (U/mg), at least about 1000 U/mg, at least about 1500 U/mg, at least about 2000 U/mg, at least about 3000 U/mg at least about 5000 U/mg, at least about 6000 U/mg, at least about 7000 U/mg, at least about 8000 U/mg, at least about 8500 U/mg, at least about 9000 U/mg, at least about 9500 U/mg, or at least about 10000 U/mg as measured by the assays described herein or any suitable assay known in the art. In some embodiments, the ALDC enzyme has an ALDC activity in the range of about 950 to 2500 units per mg of protein (U/mg), about 1000 to 2500 U/mg, or about 1500 to 2500 U/mg as measured by the assays described herein or any suitable assay known in the art. In some embodiments, the ALDC compositions according to the description comprise an ALDC enzyme with ALDC activity of at least about 900 units per gram of product, at least about 1000 U/g, at least about 1500 U/g, at least about 2000 U/g, at least about 3000 U/g at least about 5000 U/g, such as at least about 6000 U/g, such as at least about 7000 U/g, such as at least about 8000 U/g, such as at least about 8500 U/g, such as at least about 9000 U/g, such as at least about 9500 U/g, such as at least about 10000 U/g as measured by in the assays described herein or any suitable assay known in the art. In some embodiments, a different ALDC activity is used, e.g., depending on the acetolactate content and conditions requirements, e.g. for brewing. In some embodiments, the ALDC compositions according to the description comprise an ALDC enzyme with ALDC activity of at least about 8000 U/g.

In some embodiments, the compositions comprise an ALDC enzyme and a metal ion, where the metal ion is present at a concentration of about 0.1 μM to about 200 mM, such as about 1 μM to about 200 mM, or about 1 μM to about 500 μM, or about 1 μM to about 300 μM, or about 6 μM to about 300 μM, or about 10 μM to about 100 μM, or about 15 μM to about 50 μM, or about 1 μM to about 150 mM, or about 10 μM to about 150 mM, or about 20 μM to about 120 mM, or about 25 μM to about 100 mM, or about 25 μM to about 50 mM, or about 25 μM to about 20 mM, or about 25 μM to about 50 μM, or about 100 μM to about 20 mM, or about 250 μM to about 20 mM, or about 1 mM to about 20 mM, or about 1 mM to about 5 mM. In some embodiments, the compositions comprise an ALDC enzyme and a metal ion, where the metal ion is present at a concentration of about 1 μM to about 300 μM, such as about 6 μM to about 300 μM, or about 6 μM to about 50 μM, or about 6 μM to about 25 μM. In some embodiments, the compositions comprise an ALDC enzyme and a metal ion, where the metal ion is present at a concentration of about 60 μM to about 150 μM, or about 25 μM to about 150 μM. In some embodiments, the compositions comprise an ALDC enzyme and a metal ion, where the metal ion is present at a concentration of about 100 μM to about 200 mM. In some embodiments, the compositions comprise an ALDC enzyme and a metal ion, where the metal ion is present at a concentration of about 100 μM to about 20 mM. In some embodiments, the compositions comprise an ALDC enzyme and a metal ion, where the metal ion is present at a concentration of about 1 mM to about 5 mM.

In some embodiments, the compositions comprise an ALDC enzyme and zinc where the zinc is present at a concentration of about 1 μM to about 200 mM, such as about 1 μM to about 500 μM, or about 1 μM to about 300 μM, or about 6 μM to about 300 μM, or about 10 μM to about 100 μM, or about 15 μM to about 50 μM, or about 10 μM to about 150 mM, or about 20 μM to about 120 mM, or about 25 μM to about 100 mM, or about 25 μM to about 50 mM, or about 25 μM to about 20 mM, or about 25 μM to about 50 μM, or about 100 μM to about 20 mM, or about 250 μM to about 20 mM, or about 500 μM to about 20 mM, or about 1 mM to about 20 mM, or about 1 mM to about 10 mM, or about 1 mM to about 5 mM, or about 5 mM to about 20 mM, or about 5 mM to about 10 mM. In some embodiments, the compositions comprise an ALDC enzyme and zinc, where the zinc is present at a concentration of about 1 μM to about 300 μM, such about 6 μM to about 300 μM, or about 6 μM to about 25 μM. In some embodiments, the compositions comprise an ALDC enzyme and zinc, where the zinc is present at a concentration of about 25 μM to about 150 μM or about 60 μM to about 150 μM. In some embodiments, the compositions comprise an ALDC enzyme and zinc, where the zinc is present at a concentration of about 100 $\mu$M to about 20 mM. In some embodiments, the compositions comprise an ALDC enzyme and zinc, where the zinc is present at a concentration of about 100 $\mu$M to about 10 mM. In some embodiments, the compositions comprise an ALDC enzyme and zinc, where the zinc is present at a concentration of about 1 mM to about 5 mM.

In some embodiments, the compositions comprise an ALDC enzyme and zinc where the zinc is present at a concentration of about 1 mM to about 3 mM, or about 0.75 mM to about 4mM, or about 0.5 mM to about 5 mM, or about 0.25 mM to about 7.5 mM, or about 0.1 mM to about 10 mM. In some embodiments, the activity of said ALDC enzyme is in the range of 950 to 2500 Units per mg of protein, or 1000 to 2500 Units per mg of protein, or 1500 to 2500 Units per mg of protein.

In some embodiments, the compositions comprise an ALDC enzyme and zinc, where the molar ratio of zinc to enzyme is higher than 1 such as 2:1, or 3:1, or 5:1, or 10:1, or 20:1 or 30:1, or 50:1, or 60:1, or 100:1, or 150:1, or 200:1, or 250:1, or 500:1. In some embodiments, the compositions comprise an ALDC enzyme and zinc, where the molar ratio of zinc to enzyme is 2:1 or higher. In some embodiments, the compositions comprise an ALDC enzyme and zinc, where the molar ratio of zinc to enzyme is 5:1 or higher. In some embodiments, the compositions comprise an ALDC enzyme and zinc, where the molar ratio of zinc to enzyme is 10:1 or higher. In some embodiments, the compositions comprise an ALDC enzyme and zinc, where the molar ratio of zinc to enzyme is 20:1 or higher. In some embodiments, the compositions comprise an ALDC enzyme and zinc, where the molar ratio of zinc to enzyme is 30:1 or higher. In some embodiments, the compositions comprise an ALDC enzyme and zinc, where the molar ratio of zinc to enzyme is 60:1 or higher. The molar concentration of, for example, $Zn^{2+}$, $Mn^{2+}$, $Co^{2+}$ or other metal ions in solution may be determined by inductively coupled plasma optical emission spectrometry (ICP-OES) or similar techniques. The molar concentration of the ALDC enzyme may be determined using Criterion SDS-PAGE system (such as described in the examples) and the amino acid sequence.

In some embodiments, the ALDC enzyme is an ALDC derivative. In some embodiments, the ALDC derivative is an ALDC enzyme treated with glutaraldehyde. In some embodiments, the ALDC enzyme is treated with glutaraldehyde at a concentration corresponding to about 0.1 to about 5 g of glutaraldehyde per g of pure ALDC enzyme.

In some embodiments, the activity of the ALDC enzyme is in the range of 950 to 2500 Units per mg of protein. In some embodiments, the activity of said ALDC enzyme is in the range of 1000 to 2500 Units per mg of protein. In some embodiments, the activity of said ALDC enzyme is in the range of 1500 to 2500 Units per mg of protein. Thus, in some embodiments, the compositions comprise an ALDC enzyme, where the ALDC enzyme is in the range of 950 to 2500 Units per mg of protein or 1000 to 2500 Units per mg of protein or 1500 to 2500 Units per mg of protein.

In some embodiments, the compositions comprise an ALDC enzyme and zinc, where the zinc is present at a concentration of about 100 $\mu$M to about 20 mM, and the activity of said ALDC enzyme is in the range of 1000 to 2500 Units per mg of protein. In some embodiments, the compositions comprise an ALDC enzyme or an ALDC derivative and zinc, where the zinc is present at a concentration of about 250 $\mu$M to about 20 mM, and the activity of said ALDC enzyme is in the range of 1000 to 2500 Units per mg of protein.

In some embodiments, the ALDC enzyme compositions further comprise at least one additional enzyme or enzyme derivative selected from the group consisting of acetolactate reductoisomerases, acetolactate isomerases, amylase, glucoamylase, hemicellulase, cellulase, glucanase, pullulanase, isoamylase, endo-glucanase and related beta-glucan hydrolytic accessory enzymes, xylanase, xylanase accessory enzymes (for example, arabinofuranosidase, ferulic acid esterase, and xylan acetyl esterase) and protease.

In some embodiments, the ALDC enzyme compositions described herein are used during fermentation and/or maturation of a beverage preparation process, e.g., beer and wine, to reduce diacetyl levels. The terms "ALDC enzyme composition", "composition comprising an ALDC enzyme" and "composition comprising ALDC" as used herein refer to compositions comprising the ALDC enzyme. The composition may be in the form of a solution. As used herein, the terms "ALDC enzyme composition" and "compositions comprising ALDC" are mutually exclusive with media (such as cultivation media, fermentation media or maturation media) which comprise microorganisms expressing ALDC and/or capable of expressing ALDC when cultured under conditions permitting expression of the enzyme. Examples of ALDC enzyme compositions and compositions comprising ALDC include compositions comprising ALDC in a purified form. ALDC may be purified from a media comprising microorganisms capable of expressing ALDC wherein said media has been cultured under conditions permitting expression of ALDC. The term "purified" means that ALDC is present at a high level. Preferably, ALDC is the predominant component present in the composition. Preferably, ALDC is present at a level of at least about 90%, or at least about 95% or at least about 98%, said level being determined on a dry weight/dry weight basis with respect to the total composition under consideration. In some embodiments, an ALDC enzyme composition further comprises a metal ion such as zinc.

As used herein, the terms "beverage" and "beverage(s) product" include such foam forming fermented beverages as beer brewed with 100% malt, beer brewed under different types of regulations, ale, dry beer, near beer, light beer, low alcohol beer, low calorie beer, porter, bock beer, stout, malt liquor, non-alcoholic beer, non-alcoholic malt liquor and the like. The term "beverages" or "beverages product" also includes non-foaming beer and alternative malt beverages such as fruit flavored malt beverages, e. g. , citrus flavored, such as lemon-, orange-, lime-, or berry-flavored malt beverages, liquor flavored malt beverages, e. g. , vodka-, rum-, or tequila-flavored malt liquor, or coffee flavored malt beverages, such as caffeine-flavored malt liquor, and the like. The term "beverages" or "beverages product" also includes beer made with alternative materials other than malted barley, such as rye, corn, oats, rice, millet, triticale, cassava, sorghum, barley, wheat and a combination thereof. The term "beverages" or "beverages product" also includes other fermented products such as wine or ciders or perry or sake.

Beer is traditionally referred to as an alcoholic beverage derived from malt, such as malt derived from barley grain, and optionally adjunct, such as starch containing plant material (e.g. cereal grains) and optionally flavored, e.g. with hops. In the context of the present invention, the term "beer" includes any fermented wort, produced by fermentation/brewing of a starch-containing plant material, thus in particular also beer produced exclusively from adjunct, or any combination of malt and adjunct. Beer can be made from a variety of starch-containing plant material by essentially the same process, where the starch consists mainly of glucose homopolymers in which the glucose residues are linked by alpha-1, 4- or alpha-1,6-bonds, with the former predominating. Beer can be made from alternative materials such as rye, corn, oats, rice, millet, triticale, cassava, sorghum, wheat, barley and a combination thereof.

In some embodiments, the invention provides a fermentation media (e.g. beer and/or wine and/or cider and/or perry and/or sake fermentation) comprising an ALDC enzyme and metal ion at a concentration of about 1 μM to about 200 mM, such as about 1 μM to about 500 μM, or about 0.1 μM to about 300 μM, or about 1 μM to about 300 μM, or about 6 μM to about 300 μM, or about 1 μM to about 100 μM, or about 1 μM to about 50 μM, or about 6 μM to about 50 μM, or about 6 μM to about 25μM. In some embodiments, the invention provides a fermentation media (e.g. beer and/or wine and/or cider and/or perry and/or sake fermentation) comprising an ALDC enzyme and metal ion at a concentration of about 0.1 μM to about 100 mM, such as about 0.1 μM to about 10 μM, or 1 μM to about 100 mM, or 1 μM to about 10 μM, or 6 μM to about 10 μM, or about 10 μM to about 200 μM, or about 50 μM to about 1 mM, or about 100 μM to about 10 mM, or about 100 μM to about 50 mM, or about 100 μM to about 100 mM, or about 100 μM to about 200 mM, or about 250 μM to about 120 mM, or about 500 μM to about 100 mM, or about 1 mM to about 50 mM, or about 1 mM to about 20 mM, or about 1 mM to about 5 mM. In some embodiments, the invention provides a fermentation media (e.g. beer and/or wine and/or cider and/or perry and/or sake fermentation) comprising an ALDC enzyme and metal ion at a concentration of about 1 μM to about 200 mM, such as about 1 μM to about 500 μM, or about 1 μM to about 300 μM, or about 6 μM to about 300 μM, or about 1 μM to about 100 μM, or about 1 μM to about 50 μM, or about 6 μM to about 50 μM, or about 6 μM to about 25 μM. In some embodiments, the invention provides a fermentation media (e.g. beer and/or wine and/or cider and/or perry and/or sake fermentation) comprising an ALDC enzyme and metal ion at a concentration of about 1 μM to about 300 μM, or about 6 μM to about 300 μM, or about 1 μM to about 100 μM, or about 1 μM to about 50 μM, or about 6 μM to about 50 μM or about 6 μM to about 25 μM. In the media of the invention, the metal ion is $Zn^{2+}$. In some embodiments, the activity of said ALDC enzyme is in the range of 950 to 2500 Units per mg of protein, or 1000 to 2500 Units per mg of protein, or 1500 to 2500 Units per mg of protein. In some embodiments, the fermentation media (e.g. beer and/or wine and/or cider and/or perry and/or sake fermentation) further comprises at least one additional enzyme or enzyme derivative selected from the group consisting of acetolactate reductoisomerases, acetolactate isomerases, amylase, glucoamylase, hemicellulase, cellulase, glucanase, pullulanase, isoamylase, endo-glucanase and related beta-glucan hydrolytic accessory enzymes, xylanase, xylanase accessory enzymes (for example, arabinofuranosidase, ferulic acid esterase, and xylan acetyl esterase) and protease.

In some embodiments, the invention provides a maturation media (e.g. beer and/or wine and/or cider and/or perry and/or sake fermentation) comprising an ALDC enzyme and metal ion at a concentration of 1 μM to about 200 mM, such as about 1 μM to about 500 μM, or about 0.1 μM to about 300 μM, or about 1 μM to about 300 μM, or about 6 μM to about 300 μM, or about 1 μM to about 100 μM, or about 1 μM to about 50 μM, or about 6 μM to about 50 μM, or about 6 μM to about 25μM. In some embodiments, the invention provides a maturation media (e.g. beer and/or wine and/or cider and/or perry and/or sake fermentation) comprising an ALDC enzyme and metal ion at a concentration of about 0.1 μM to about 100 mM, or 1 μM to about 100 mM, such as about 0.1 μM to about 10 μM, or 1 μM to about 10 μM, or 6 μM to about 10 μM, or about 10 μM to about 200 μM, or about 50 μM to about 1 mM, or about 100 μM to about 10 mM, or about 100 μM to about 50 mM, or about 100 μM to about 100 mM, or about 100 μM to about 200 mM, or about 250 μM to about 120 mM, or about 500 μM to about 100 mM, or about 1 mM to about 50 mM, or about 1 mM to about 20 mM, or about 1 mM to about 5 mM. In some embodiments, the invention provides a maturation media (e.g. beer and/or wine and/or cider and/or perry and/or sake fermentation) comprising an ALDC enzyme and metal ion at a concentration of about 1 μM to about 500 μM, or about 1 μM to about 300 μM, or about 6 μM to about 300 μM, or about 1 μM to about 100 μM, or about 1 μM to about 50 μM, or about 6 μM to about 50 μM, or about 6 μM to about 25 μM. In some embodiments, the invention provides a maturation media (e.g. beer and/or wine and/or cider and/or perry and/or sake fermentation) comprising an ALDC enzyme and metal ion at a concentration of about 1 μM to about 300 μM, or about 6 μM to about 300 μM, or about 1 μM to about 100 μM, or about 1 μM to about 50 μM, or about 6 μM to about 50 μM, or about 6 μM to about 25 μM. In the media of the invention, the metal ion is $Zn^{2+}$. In some embodiments, the activity of said ALDC enzyme is in the range of 950 to 2500 Units per mg of protein, or 1000 to 2500 Units per mg of protein, or 1500 to 2500 Units per mg of protein. In some embodiments, the maturation media (e.g. beer and/or wine maturation) further comprises at least one additional enzyme or enzyme derivative selected from the group consisting of acetolactate reductoisomerases, acetolactate isomerases, amylase, glucoamylase, hemicellulase, cellulase, glucanase, pullulanase, isoamylase, endo-glucanase and related beta-glucan hydrolytic accessory enzymes, xylanase, xylanase accessory enzymes (for example, arabinofuranosidase, ferulic acid esterase, and xylan acetyl esterase) and protease.

In some embodiments, metal ions such as $Zn^{2+}$, $Mg^{2+}$, $Mn^{2+}$, $Co^{2+}$, $Cu^{2+}$, $Ba^{2+}$, $Ca^{2+}$ and $Fe^{2+}$ and combinations thereof are added to the cultivation and/or fermentation media during and/or after ALDC production to increase the recovered yields from microorganisms.

The term "cultivation media" as used herein refers to a media which supports the growth of microorganisms such as an ALDC producing host cell. Examples of a cultivation media include: media based on MOPs buffer with, for instance, urea as the major nitrogen source and maltrin as the main carbon source; and TSB broth.

Materials may be added to an enzyme-containing composition to improve the properties of the composition. Non-limiting examples of such additives include: salts (e.g., alkali salts, earth metal salts, additional chloride salts, sulfate salts, nitrate salts, carbonate salts, where exemplary counter ions are calcium, potassium, and sodium), inorganic minerals or clays (e.g., zeolites, kaolin, bentonite, talcs and/or silicates), carbohydrates (e.g., sucrose and/or starch), coloring pigments (e.g., titanium dioxide), biocides (e.g., Rodalon®, Proxel®), dispersants, anti-foaming agents, reducing agents, acid agents, alkaline agents, enzyme stabilizers (e.g. polyol such as glycerol, propylene glycol, sorbitol, inorganic salts, sugars, sugar or a sugar alcohol, lactic acid, boric acid, or a boric acid derivative and combinations thereof), enzyme inhibitors, preservative (e.g. methyl paraben, propyl paraben, benzoate, sorbate or other food approved preservatives) and combinations thereof. Excipients which may be used in the composition, or the preparation thereof, include maltose, maltose syrup, sucrose, glucose (including glucose syrup or dried glucose syrup), pre-cooked starch, gelatinised starch, L-lactic, ascorbyl palmitate, tocopherols, lecithins, citric acid, citrates, phosphoric, phosphates, sodium alginate, carrageenan, locust bean gum, guar gum, xanthan gum, pectins, sodium carboxymethylcellulose, mono- and diglycerides, citric acid esters of mono- and diglycerides, sucrose esters, carbon dioxide, argon, helium, nitrogen, nitrous oxide, oxygen, hydrogen, and starch sodium octenylsuccinate.

**Methods**

In some aspects the invention provides methods to improve stability and/or activity of ALDC enzymes.

In some embodiments, the description provides methods for increasing the activity and/or stability of an ALDC enzyme in a composition comprising ALDC wherein said method comprises the step of adding a metal ion to the composition so that said metal ion is present in said composition at a concentration of about 1 μM to about 200 mM, such as about 1 μM to about 500 μM, or about 1 μM to about 300 μM, or about 6 μM to about 300 μM, or about 1 μM to about 100 μM, or about 1 μM to about 50 μM, or about 10 μM to about 150 mM, or about 20 μM to about 120 mM, or about 25 μM to about 100 mM, or about 25 μM to about 50 mM, or about 25 μM to about 20 mM, or about 25 μM to about 50 μM, or about 100 μM to about 20 mM, or about 250 μM to about 20 mM, or about 500 μM to about 20 mM, or about 1 mM to about 20 mM, or about 1 mM to about 10 mM, or about 1 mM to about 5 mM, or about 5 mM to about 20 mM, or about 5 mM to about 10 mM. In the methods of the invention, the metal ion is $Zn^{2+}$.

In some embodiments, the invention provides methods for increasing the activity and/or stability of an ALDC enzyme in a fermentation and/or maturation media comprising an ALDC enzyme wherein said method comprises the step of adding zinc to the media so that said zinc is present in said media at a concentration of about 1 μM to about 300 μM, such as about 6 μM to about 300 μM, about 1 μM to about 100 μM, about 1 μM to about 50 μM, about 1 μM to about 25 μM, or about 6 μM to about 25 μM. In some embodiments, methods for increasing the activity and/or stability of an ALDC enzyme comprise adding a zinc to a media so that the zinc is at a concentration of about 25 μM to about 150 μM in the media. In some embodiments, methods for increasing the activity and/or stability of an ALDC enzyme comprise adding zinc at a concentration of about 100 μM to about 20 mM. In some embodiments, methods for increasing the activity and/or stability of an ALDC enzyme comprise adding zinc at a concentration of about 1 mM to about 5 mM. In some embodiments, methods for increasing the activity and/or stability of an ALDC enzyme comprise adding zinc at a molar ratio of zinc to ALDC enzyme that is higher than 1 such as 2:1, or 3:1, or 5:1, or 10:1, or 20:1 or 30:1, or 50:1, or 60:1, or 100:1, or 150:1, or 200:1 or 250:1 in said composition. In some embodiments, methods for increasing the activity and/or stability of an ALDC enzyme comprise adding zinc at a molar ratio of zinc to ALDC enzyme of 5:1 or higher in said composition. In some embodiments, methods for increasing the activity and/or stability of an ALDC enzyme comprise adding zinc at a molar ratio of zinc to ALDC enzyme of 10:1 or higher in said composition. In some embodiments, methods for increasing the activity and/or stability of an ALDC enzyme comprise adding zinc at a molar ratio of zinc to ALDC enzyme of 20:1 or higher in said composition. In some embodiments, methods for increasing the activity and/or stability of an ALDC enzyme comprise adding zinc at a molar ratio of zinc to ALDC enzyme of 30:1 or higher in said composition. In some embodiments, the metal ion is added (e.g. as a supplement) to a cultivation media during the production of said ALDC enzyme by an ALDC producing host cell. In some embodiments, the metal ion is added at a concentration of about 0.1 μM to about 1 mM, such as about 25 μM to about 150 μM, or about 40 μM to about 150 μM, or about 60 μM to about 150 μM, or about 25 μM to about 50 μM, or 30 μM to about 40 μM. In the methods of the invention, the metal ion is $Zn^{2+}$. Thus, in some embodiments zinc is added (e.g. as a supplement) to a cultivation media during the production of said ALDC enzyme by an ALDC producing host cell at a concentration of 1 μM to about 1 mM, such as 25 μM to about 150 μM, or about 40 μM to about 150 μM, or 60 μM to about 150 μM.

In some embodiments, the host cell is a *Bacillus* host cell. In some embodiments, Bacillus host cell is *Bacillus subtilis.*

The metal ion is added in the fermentation media during beer and/or wine and/or cider and/or perry and/or sake fermentation. In the methods of the invention, the metal ion is $Zn^{2+}$. Thus, zinc is added in a fermentation media during beer and/or wine and/or cider and/or perry and/or sake fermentation. In some embodiments, zinc is added at a concentration of about 1 μM to about 300 μM, or about 6 μM to about 300 μM, or about 1 μM to about 100 μM, or 25 μM to about 50 μM, or 30 μM to about 40 μM, or 1 μM to about 50 μM, or 6 μM to about 50 μM, or 1 μM to about 25 μM, or 6 μM to about 25 μM. In some embodiments zinc and the ALDC enzyme are added in a composition, wherein the molar ratio of zinc to ALDC enzyme in the composition is higher than 1 such as 2:1, or 3:1, or 5:1, or 10:1, or 20:1 or 30:1, or 50:1, or 60:1.

The metal ion is added the maturation media during beer and/or wine and/or cider and/or perry and/or sake fermentation. In the methods of the invention, the metal ion is $Zn^{2+}$. Thus, zinc is added in a maturation media during beer and/or wine and/or cider and/or perry and/or sake fermentation. In some embodiments, zinc is added at a concentration of 1 μM to about 300 μM, or about 6 μM to about 300 μM, or about 1 μM to about 100 μM, or 25 μM to about 50 μM, or 30 μM to about 40 μM, or 1 μM to about 50 μM, or 6 μM to about 50 μM, or 1 μM to about 25 μM, or 6 μM to about 25 μM. In some embodiments zinc and the ALDC enzyme are added in a composition, wherein the molar ratio of zinc to ALDC enzyme in the composition is higher than 1 such as 2:1, or 3:1, or 5:1, or 10:1, or 20:1 or 30:1, or 50:1, or 60:1.

*Fermented Products*

As used herein, a fermented alcoholic product with "low diacetyl content" refers to a fermented alcoholic product (e.g. a beer and/or a wine and/or a cider and/or a perry and/or sake) produced by fermentation of a carbohydrate containing substrate with a composition comprising ALDC in the presence of a metal ion (such as zinc) wherein the diacetyl levels are lower when compared to the fermented alcoholic produced by fermentation of a carbohydrate containing substrate with a composition comprising ALDC in the absence of a metal ion (such as zinc) under the same fermentation conditions (e.g. same temperature and for the same length of time). Examples of fermented alcoholic products with low diacetyl content are fermented alcoholic products in which the levels of diacetyl are less than about 1 ppm and/or the diacetyl levels are below about 0.5 mg/L. Suitably, the diacetyl levels are less than about 0.5 ppm, or less than about 0.1 ppm, or less than about 0.05 ppm, or less than about 0.01 ppm, or less than about 0.001 ppm. In one embodiment, the diacetyl levels are about less than 0.1 mg/L, or about less than 0.05 mg/L, or about less than 0.01 mg/L or about less than 0.001 mg/L.

When carbohydrate containing substrates, such as wort (e.g. worts with low malt content) or fruit juices (such as grape juice, apple juice or pear juice), are fermented with yeast or other microorganisms, various processes take place in addition to the alcohol fermentation which may cause generation of undesired by-products, e.g., the formation of diacetyl which has a strong and unpleasant smell even in very low concentrations. Alcoholic beverages, such as beer or wine or cider or perry or sake, may thus have an unacceptable aroma and flavor if the content of diacetyl considerably exceeds certain limits, e.g., in the case of some beers about 0.1 ppm.

Formation of diacetyl is also disadvantageous in the industrial production of ethanol because it is difficult to separate diacetyl from ethanol by distillation. A particular problem arises in the preparation of absolute ethanol where ethanol is dehydrated by azeotropic distillation with benzene. Diacetyl will accumulate in the benzene phase during the azeotropic distillation which may give rise to mixtures of diacetyl and benzene which makes it difficult to recover the benzene used for the azeotropic distillation.

The conventional brewing of beer comprises fermenting the wort with a suitable species of yeast, such as *Saccharomyces cerevisae* or *Saccharomyces carlsbergensis.*

Typically in conventional brewing, the fermentation is usually effected in two steps, a main fermentation of a duration of normally 5 to 12 days and a secondary fermentation - a so-called maturation process-which may take from up to 12 weeks. During the main fermentation most of the carbohydrates in the wort are converted to ethanol and carbon dioxide. Maturation is usually effected at a low temperature in the presence of a small residual amount of yeast. The purposes of the maturation are, inter alia, to precipitate undesirable, high molecular weight compounds and to convert undesirable compounds to compounds, such as diols, which do not affect flavor and aroma. For example butanediol, the final product of the conversion of α-acetolactate and diacetyl in beer, is typically reported as a compound with neutral sensory characteristics. The term "fermentation media" as used herein refers to a medium comprising carbohydrate containing substrates which can be fermented by yeast or other microorganisms to produce, for example, beer or wine or cider or perry or sake. Examples of fermentation media include: wort, and fruit juices (such as grape juice, apple juice and pear juice). Example 9 details an example of suitable wort. The term "maturation media" as used herein refers to a medium comprising carbohydrate containing substrates which have been fermented by yeast or other microorganisms to produce, for example, beer or wine or cider or perry or sake. Examples of maturation media include partially fermented wort and fruit juices (such as grape juice, apple juice and pear juice). In some embodiments, the invention provides a fermentation or maturation media for an ALDC producing host cell comprising a metal ion at a concentration of about 1 $\mu$M to about 300 $\mu$M. In some embodiments, the invention provides a fermentation or maturation media for an ALDC producing host cell comprising a metal ion at a concentration of about 6 $\mu$M to about 25 $\mu$M.

In some embodiments, the methods of the invention are thus characterized by the treatment of a substrate with a composition comprising ALDC or an ALDC derivative as described herein during or in continuation of a fermentation process, e.g., maturation.

Thus, in some embodiments, acetolactate is enzymatically decarboxylated to acetoin, the result being that when undesirable, the formation of diacetyl from acetolactate is avoided. In some embodiments, other enzymes are used in combination with ALDC for the conversion of $\alpha$-acetolactate. Examples of such enzymes include, but are not limited to, acetolactate reductoisomerases or isomerases.

In some embodiments, the ALDC and/or ALDC derivative compositions described herein are used together with ordinary yeast in batch fermentation.

Instead of using the enzyme in a free state, it may be used in an immobilized state, the immobilized enzyme being added to the wort during or in continuation of the fermentation (e.g., during maturation). The immobilized enzyme may also be maintained in a column through which the fermenting wort or the beer is passed. The enzyme may be immobilized separately, or coimmobilized yeast cells and acetolactate decarboxylase may be used.

In some embodiments, the ALDC and/or ALDC derivative compositions described herein are used during beer and/or wine and/or cider and/or perry and/or sake fermentation or maturation to reduce the diacetyl levels to below about 1 ppm, or about less than 0.5 ppm, or about less than 0.1 ppm, or about less than 0.05 ppm or about less than 0.01 ppm, or about less than 0.001 ppm. In some embodiments, the ALDC and/or ALDC derivative compositions described herein are used during beer and/or wine and/or cider and/or perry and/or sake fermentation or maturation to reduce the diacetyl levels to below 0.1 ppm.

In some embodiments, the ALDC and/or ALDC derivative compositions described herein are used during beer and/or wine and/or cider and/or perry and/or sake fermentation or maturation to reduce VDK content below 0.1 mg/L, or about less than 0.05 mg/L, or less than 0.01 mg/L or less than 0.001 mg/L. Total VDK refers to the amount of Diacetyl plus 2,3-pentanedione. In some embodiments, the ALDC and/or ALDC derivative compositions described herein are used during beer and/or wine and/or cider and/or perry and/or sake fermentation or maturation to reduce Total VDK content below 0.1 mg/L.

In some embodiments, the ALDC and/or ALDC derivative compositions described herein are used during beer and/or wine and/or cider and/or perry and/or sake fermentation or maturation to reduce the diacetyl levels to below about 0.5 mg/L, or about less than 0.1 mg/L, or about less than 0.05 mg/L, or about less than 0.01 mg/L or about less than 0.001 mg/L. In some embodiments, the ALDC and/or ALDC derivative compositions described herein are used during beer and/or wine and/or cider and/or perry and/or sake fermentation or maturation to reduce the diacetyl levels to below 0.1 mg/L.

In some embodiments, the ALDC and/or ALDC derivative compositions described herein are used during beer and/or wine and/or cider and/or perry and/or sake fermentation or maturation to reduce other vicinal diketones (such as 2,3 pentanedione) to below 0.1 mg/L, or about less than 0.05 mg/L, or less than 0.01 mg/L or less than 0.001 mg/L.

The processes of the invention can not only be used in connection with the brewing of beer, but is also suitable for the production of any suitable alcoholic beverage where a reduction in diacetyl levels or other vicinal diketones is desirable (e.g. wine, sake, cider, perry, etc.). In some embodiments, the processes of the invention can be used in the production of wine where similar advantages are obtained, in particular a reduction in the maturation period and a simplification of the process. Of special interest in this context is the use of acetolactate converting enzymes in connection with the so-called malo-lactic fermentation. This process which is affected by microorganisms as species of *Leuconostoc, Lactobacillus* or *Pediococcus* is carried out after the main fermentation of wine in order to increase the pH of the product as well as its biological stability and to develop the flavor of the wine. Moreover, it is highly desirable to carry out the fermentation since it makes possible rapid bottling and thereby improves the cash-flow of wineries substantially. Unfortunately, however, the process may give rise to off-flavors due to diacetyl, the formation of which can be reduced with the aid of acetolactate converting enzymes.

Thus, in some embodiments, the processes of the invention provide for the production of alcoholic beverages with lower content of diacetyl, wherein the time required for producing the alcoholic beverages with lower content of diacetyl is reduced by at least 10%, or at least 20% or at least 30%, or at least 40%, or at least 50%, or at least 60%, or at least 70%, or at least 80%, or at least 90% when compared to a process without the use of the ALDC and/or ALDC derivative compositions described herein. In some embodiments, the processes of the invention provide for the production of alcoholic beverages with lower content of diacetyl when compared to a process without the use of the ALDC and/or ALDC derivative compositions described herein, wherein a maturation step is completely eliminated.

In some embodiments, the ALDC and/or ALDC derivative compositions described herein are used during a fermentation process (e.g. beer and/or wine and/or cider and/or perry and/or sake fermentation), such that the time required for the fermentation process is reduced by at least 10%, or at least 20% or at least 30%, or at least 40%, or at least 50%, or at least 60%, or at least 70%, or at least 80%, or at least 90%, when compared to a process without the use of the ALDC and/or ALDC derivative compositions described herein. In some embodiments, the processes of the invention provide for the production of alcoholic beverages with lower content of diacetyl when compared to a process without the use of the ALDC and/or ALDC derivative compositions described herein, wherein a maturation step is completely eliminated.

In some embodiments, the ALDC and/or ALDC derivative compositions described herein are used during a maturation or conditioning process (e.g. beer maturation/conditioning), such that the time required for the maturation or conditioning process is reduced by at least 10%, or at least 20% or at least 30%, or at least 40%, or at least 50%, or at least 60%, or at least 70%, or at least 80%, or at least 90%, when compared to a process without the use of the ALDC and/or ALDC derivative compositions described herein. In some embodiments, the processes of the invention provide for the production of alcoholic beverages with lower content of diacetyl when compared to a process without the use of the ALDC and/or ALDC derivative compositions described herein, wherein a maturation step is completely eliminated.

Further, in some embodiments, the processes described herein can be used to advantage for industrial preparation of ethanol as fermentation products are obtained without or practically without any content of diacetyl, which simplifies the distillation process, especially in case of azeotropic for the preparation of absolute ethanol, i.e. pure anhydrous ethanol.

In some embodiments, the ALDC enzyme and said zinc are added during a fermentation process and/or a maturation process. In some embodiments, the ALDC enzyme is added at a concentration of about 0.01 g to about 10 g, or about 0.5 g to about 10 g, or about 1 g to about 5 g per hectoliter of beer and/or wine and/or cider and/or perry and/or sake ferment. As used herein the term "beer and/or wine and/or cider and/or perry and/or sake ferment" may be interchangeable with the term media. In some embodiments, the activity of said ALDC enzyme is in the range of 1000 to 2500 Units per mg of protein.

**Production of ALDC enzymes**

In a further aspect, the host cell is a fungal cell. In yet a further aspect, the fungal cell is of the genus *Trichoderma.* In yet a further aspect, the fungal cell is of the species *Trichoderma reesei* or of the species *Hypocrea jecorina.* In another aspect, the host cell comprises, preferably is transformed with, a plasmid or an expression vector as described herein.

In some embodiments, the host cell is a bacterial host cell such as *Bacillus.* In some embodiments the ALDC enzyme is produced by cultivation of a *Bacillus subtilis* strain containing a gene encoding and expressing an ALDC enzyme (e.g. ALDC of *Bacillus brevis*). Examples of such host cells and cultivation thereof are described in DK149335B.

Examples of suitable expression and/or integration vectors are provided in Sambrook et al. (1989) *supra,* and Ausubel (1987) *supra,* and van den Hondel et al. (1991) in Bennett and Lasure (Eds.) More Gene Manipulations In Fungi, Academic Press pp. 396-428 and U.S. Patent No. 5,874,276. Reference is also made to the Fungal Genetics Stock Center Catalogue of Strains (FGSC, http://www.fgsc.net) for a list of vectors. Particularly useful vectors include vectors obtained from for example Invitrogen and Promega. Suitable plasmids for use in bacterial cells include pBR322 and pUC19 permitting replication in *E. coli* and pE194 for example permitting replication in *Bacillus.* Other specific vectors suitable for use in *E. coli* host cells include vectors such as pFB6, pBR322, pUC18, pUC100, pDONR™201, 10 pDONR™221, pENTR™, pGEM®3Z and pGEM®4Z.

Specific vectors suitable for use in fungal cells include pRAX, a general purpose expression vector useful in *Aspergillus,* pRAX with a *glaA* promoter, and in *Hypocrea*/*Trichoderma* includes pTrex3g with a *cbh*1 promoter.

In some embodiments, the host cells are fungal cells and optionally filamentous fungal host cells. The term "filamentous fungi" refers to all filamentous forms of the subdivision Eumycotina (*see*, Alexopoulos, C. J. (1962), Introductory Mycology, Wiley, New York). These fungi are characterized by a vegetative mycelium with a cell wall composed of chitin, cellulose, and other complex polysaccharides. The filamentous fungi of the present disclosure are morphologically, physiologically, and genetically distinct from yeasts. Vegetative growth by filamentous fungi is by hyphal elongation and carbon catabolism is obligatory aerobic. In the present disclosure, the filamentous fungal parent cell may be a cell of a species of, but not limited to, *Trichoderma* (*e.g., Trichoderma reesei*, the asexual morph of *Hypocrea jecorina*, previously classified as *T. longibrachiatum, Trichoderma viride, Trichoderma koningii, Trichoderma harzianum*) (Sheir-Neirs et al., Appl. Microbiol. Biotechnol. 20:46-53 (1984); ATCC No. 56765 and ATCC No. 26921), *Penicilliurn sp., Humicola sp.* (*e.g., H. insolens, H. lanuginosa* and *H. grisea*), *Chrysosporium sp.* (*e.g., C. lucknowense*), *Gliocladium*

*sp., Aspergillus sp.* (*e.g., A. oryzae, A. niger, A sojae, A. japonicus, A. nidulans,* and *A. awamori*) (Ward et al., Appl. Microbiol. Biotechnol. 39:738-743 (1993) and Goedegebuur et al., Curr. Genet. 41:89 -98 (2002)), *Fusarium sp.*,(*e.g., F. roseum, F. graminum, F. cerealis, F. oxysporum,* and *F. venenatum*), *Neurospora sp.,* (*N. crassa*), *Hypocrea sp., Mucor sp.* (*M. miehei*), *Rhizopus sp.,* and *Emericella sp.* (*see also,* Innis et al., Science 228:21 -26 (1985)). The term "*Trichoderma*" or "*Trichoderma sp.*" or "*Trichoderma spp.*" refer to any fungal genus previously or currently classified as *Trichoderma.*

In some embodiments, the host cells will be gram-positive bacterial cells. Non-limiting examples include strains of *Streptomyces* (*e.g., S. lividans, S. coelicolor,* and *S. griseus*) and *Bacillus.* As used herein, "the genus *Bacillus*" includes all species within the genus "*Bacillus*," as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named "*Geobacillus tearothermophilus*."

In some embodiments, the host cell is a gram-negative bacterial strain, such as *E. coli* or *Pseudomonas sp.* In other embodiments, the host cells may be yeast cells such as *Saccharomyces sp., Schizosaccharomyces sp., Pichia sp.,* or *Candida sp.* In other embodiments, the host cell will be a genetically engineered host cell wherein native genes have been inactivated, for example by deletion in bacterial or fungal cells. Where it is desired to obtain a fungal host cell having one or more inactivated genes known methods may be used (*e.g.*, methods disclosed in U.S. Patent No. 5,246,853, U.S. Patent No. 5,475,101, and WO 92/06209). Gene inactivation may be accomplished by complete or partial deletion, by insertional inactivation or by any other means that renders a gene nonfunctional for its intended purpose (such that the gene is prevented from expression of a functional protein). In some embodiments, when the host cell is a *Trichoderma* cell and particularly a *T. reesei* host cell, the *cbh*1*, cbh*2, *egl*1 and *egl*2 genes will be inactivated and/or deleted. Exemplary *Trichoderma reesei* host cells having quad-deleted proteins are set forth and described in U.S. Patent No. 5,847,276 and WO 05/001036. In other embodiments, the host cell is a protease deficient or protease minus strain. The term "protease deficient" or a "protease minus strain" as used herein refers to a host cell derived or derivable from a parental cell wherein the host cell comprises one or more genetic alterations that causes the host cells to produce a decreased amount of one or more proteases (e.g. functional proteases) when compared to the parental cell; preferably said host cell is deficient in one or more proteases selected from the group consisting of WprA, Vpr, Epr, IspA, Bpr, NprE, AprE, ampS, aprX, bpf, clpCP, clpEP, clpXP, codWX, lonA, lonB, nprB, map, mlpA, mpr, pepT, pepF, dppA, yqyE, tepA, yfiT, yflG, ymfF, ypwA, yrrN, yrrO, and ywaD. a variant host cell derived from a parental cell is provided, the variant host cell comprises one or more genetic alterations that causes cells of the variant strain to produce a decreased amount of one or more proteases when compared to the parental cell.

Introduction of a DNA construct or vector into a host cell includes techniques such as transformation; electroporation; nuclear microinjection; transduction; transfection, (*e.g.*, lipofection-mediated and DEAE-Dextrin mediated transfection); incubation with calcium phosphate DNA precipitate; high velocity bombardment with DNA-coated microprojectiles; and protoplast fusion. General transformation techniques are known in the art (*see, e.g.,* Ausubel et al. (1987) *supra,* chapter 9; and Sambrook et al. (1989) *supra,* and Campbell et al., Curr. Genet. 16:53-56 (1989)).

Transformation methods for *Bacillus* are disclosed in numerous references including Anagnostopoulos C. and J. Spizizen, J. Bacteriol. 81:741-746 (1961) and WO 02/14490.

Transformation methods for *Aspergillus* are described in Yelton et al., Proc. Natl. Acad. Sci. USA 81:1470-1474 (1984); Berka et al., (1991) in Applications of Enzyme Biotechnology, Eds. Kelly and Baldwin, Plenum Press (NY); Cao et al., Protein Sci. 9:991-1001 (2000); Campbell et al., Curr. Genet. 16:53-56 (1989), and EP 238 023. The expression of heterologous protein in *Trichoderma* is described in U.S. Patent No. 6,022,725; U.S. Patent No. 6,268,328; Harkki et al. Enzyme Microb. Technol. 13:227-233 (1991); Harkki et al., BioTechnol. 7:596-603 (1989); EP 244,234; EP 215,594; and Nevalainen et al., "The Molecular Biology of Trichoderma and its Application to the Expression of Both Homologous and Heterologous Genes", in Molecular Industrial Mycology, Eds. Leong and Berka, Marcel Dekker Inc., NY (1992) pp. 129-148). Reference is also made to WO96/00787 and Bajar et al., Proc. Natl. Acad. Sci. USA 88:8202-8212 (1991) for transformation of *Fusarium* strains.

The term "ALDC producing host cell" as used herein refers to a host cell capable of expressing ALDC enzyme when said host cell is cultured under conditions permitting the expression of the nucleic acid sequence encoding ALDC. The nucleic acid sequence encoding ALDC enzyme may be heterologous or homologous to the host cell. In some embodiments, the ALDC producing host cell is *Bacillus subtilis.* In some embodiments, the ALDC producing host cell is *Bacillus subtilis* comprising a gene encoding and expressing ALDC enzyme wherein the ALDC enzyme comprises an amino acid sequence having at least 70%, 75%, 80%, 85%, 90%, 95%, 97%, 98%, 99% or 100% identity with SEQ ID NO: 2, SEQ ID NO: 3, SEQ ID NO: 5, SEQ ID NO: 7, SEQ ID NO: 8, or any functional fragment thereof. In some embodiments, the ALDC producing host cell is *Bacillus subtilis* comprising a nucleic acid sequence encoding ALDC wherein said nucleic acid sequence encoding ALDC has at least 70%, 75%, 80%, 85%, 90%, 95%, 97%, 98%, 99% or 100% identity with SEQ ID NO: 1, SEQ ID NO: 4, SEQ ID NO: 6 or any functional fragment thereof. In some embodiments, the ALDC producing host cell is *Bacillus subtilis* comprising a gene encoding ALDC derived from *Bacillus brevis.*

## EXAMPLES

The present disclosure is described in further detail in the following examples, which are not in any way intended to limit the scope of the disclosure as claimed. The attached figures are meant to be considered as integral parts of the specification and description of the disclosure. The following examples are offered to illustrate, but not to limit the claimed disclosure.

### Example 1 - Heterologous expression of acetolactate decarboxylase, aldB

The *Brevibacillus brevis* (which may be referred to as *Bacillus brevis*) acetolactate decarboxylases (ALDC) *aldB* gene was previously identified (Diderichsen et al., J Bacteriol. (1990) 172(8): 4315), with the sequence set forth as UNIPROT Accession No. P23616.1. The sequence of this gene, *aldB*, is depicted in SEQ ID NO:1. The nucleotides highlighted in bold and underlined are the nucleotides which encode the signal peptide.

*SEQ ID NO: 1 sets forth the nucleotide sequence of the aldB gene:*

**atgaaaaaaaatatcatcacttctatcacatctctggctctggttgccgggctgtctttgactgcttttgca**gctacaacggctactgtac
cagcaccacctgccaagcaggaatccaaacctgcggttgccgctaatccggcaccaaaaaatgtactgtttcaatactcaacgatcaatgca
ctcatgcttggacagtttgaaggggacttgactttgaaagacctgaagctgcgaggcgatatggggcttggtaccatcaatgatctcgatgga
gagatgattcagatgggtacaaaattctaccagatcgacagcaccggaaaattatcggagctgccagaaagtgtgaaaactccatttgcggt
tactacacatttcgagccgaaagaaaaaactacattaaccaatgtgcaagattacaatcaattaacaaaaatgcttgaggagaaatttgaaaac
aagaacgtcttttatgccgtaaagctgaccggtacctttaagatggtaaaggctagaacagttccaaaacaaaccagaccttatccgcagctg
actgaagtaaccaaaaaacaatccgagtttgaatttaaaaatgttaagggaaccctgattggcttctatacgccaaattatgcagcagccctga
atgttcccggattccatctccacttcatcacagaggataaaacaagtggcggacacgtattaaatctgcaatttgacaacgcgaatctggaaat
ttctccgatccatgagtttgatgtacaattgccgcacacagatgattttgcccactctgatctgacacaagttactactagccaagtacaccaag
ctgagtcagaaagaaaataa

*SEQ ID NO: 6 sets forth an example of a nucleotide sequence encoding an acetolactate decarboxylase* - the nucleotides highlighted in bold and underlined are the nucleotides which encode the signal peptide:

**atgaaaaaaaatatcatcacttctatcacatctctggctctcgttgccgggctgtctttgactgcttttgcagctacaacggctactgta
ccagcaccacctgccaagcaggaatccaaacctgtggttgccgctaatccggcaccaaaaaatgtactgtttcaatactcaacgat
caatgcactcatgcttggacagtttgaaggggacttgactttgaaagacctgaagctacgaggcgatatggggcttggt**accatcaa
tgatctcgatggagagatgattcagatgggtacaaaattctaccagatcgacagcaccggaaaattatccgagctgccagaaagtgtgaaaa
ctccatttgcggttactacacatttcgagccgaaagaaaaaactacattaaccaatgtgcaagattacaatcaattaacaaaaatgcttgagga
gaaatttgaaaacaagaacgtcttttatgccgtaaagctgaccggtacctttaagatggtaaaggctagaacagttccaaaacaaaccagacc
ttatccgcagctgactgaagtaaccaaaaaacaatccgagtttgaatttaaaaatgttaagggaaccctgattggcttctatacgccaaattatg
cagcagccctgaatgttcccggattccatctccacttcatcacagaggataaaacaagtggcggacacgtattaaatctgcaatttgacaacg
cgaatctggaaatttctccgatccatgagtttgatgtacaattgccgcacacagatgattttgcccactctgatctgacacaagttactactagcc
aagtacaccaagctgagtcagaaagaaaataa

The proenzyme encoded by the *aldB* gene is depicted in SEQ ID NO: 2. At the N-terminus, the protein has a signal peptide with a length of 24 amino acids as predicted by SignalP-NN (Emanuelsson et al., Nature Protocols (2007) 2: 953-971). This signal peptide sequence is underlined and is in bold in SEQ ID NO:2. The presence of a signal peptide indicates that this acetolactate decarboxylase, aldB is a secreted enzyme. The sequence of the predicted, fully processed mature chain (aldB, 261 amino acids) is depicted in SEQ ID NO: 3.

*SEQ ID NO: 2 sets forth the amino acid sequence of the acetolactate decarboxylase (ALDC) precursor aldB*:

**MKKNIITSITSLALVAGLSLTAFA**ATTATVPAPPAKQESKPAVAANPAPKNVLFQYSTI
NALMLGQFEGDLTLKDLKLRGDMGLGTINDLDGEMIQMGTKFYQIDSTGKLSELPESV
KTPFAVTTHFEPKEKTTLTNVQDYNQLTKMLEEKFENKNVFYAVKLTGTFKMVKARTV
PKQTRPYPQLTEVTKKQSEFEFKNVKGTLIGFYTPNYAAALNVPGFHLHFITEDKTSGGH
VLNLQFDNANLEISPIHEFDVQLPHTDDFAHSDLTQVTTSQVHQAESERK

*SEQ ID NO: 7 sets forth an example of an amino acid sequence of the acetolactate decarboxylase* (*ALDC*) *precursor aldB* - the signal peptide *sequence* is underlined and is in bold:

**MKKNIITSITSLALVAGLSLTAFA**ATTATVPAPPAKQESKPVVAANPAPKNVLFQYSTI
NALMLGQFEGDLTLKDLKLRGDMGLGTINDLDGEMIQMGTKFYQIDSTGKLSELPESV
KTPFAVTTHFEPKEKTTLTNVQDYNQLTKMLEEKFENKNVFYAVKLTGTFKMVKARTV
PKQTRPYPQLTEVTKKQSEFEFKNVKGTLIGFYTPNYAAALNVPGFHLHFITEDKTSGGH
VLNLQFDNANLEISPIHEFDVQLPHTDDFAHSDLTQVTTSQVHQAESERK

*SEQ ID NO: 3 sets forth the predicted amino acid sequence of the mature acetolactate decarboxylase (ALDC) aldB (261 amino acids):*

ATTATVPAPPAKQESKPAVAANPAPKNVLFQYSTINALMLGQFEGDLTLKDLKLRGDM
GLGTINDLDGEMIQMGTKFYQIDSTGKLSELPESVKTPFAVTTHFEPKEKTTLTNVQDYN
QLTKMLEEKFENKNVFYAVKLTGTFKMVKARTVPKQTRPYPQLTEVTKKQSEFEFKNV
KGTLIGFYTPNYAAALNVPGFHLHFITEDKTSGGHVLNLQFDNANLEISPIHEFDVQLPHT
DDFAHSDLTQVTTSQVHQAESERK

*SEQ ID NO: 8 sets forth an example of the predicted amino acid sequence of the mature acetolactate decarboxylase (ALDC) aldB (261 amino acids):*

ATTATVPAPPAKQESKPVVAANPAPKNVLFQYSTINALMLGQFEGDLTLKDLKLRGDM
GLGTINDLDGEMIQMGTKFYQIDSTGKLSELPESVKTPFAVTTHFEPKEKTTLTNVQDYN

QLTKMLEEKFENKNVFYAVKLTGTFKMVKARTVPKQTRPYPQLTEVTKKQSEFEFKNV
KGTLIGFYTPNYAAALNVPGFHLHFITEDKTSGGHVLNLQFDNANLEISPIHEFDVQLPHT
DDFAHSDLTQVTTSQVHQAESERK

The *aldB* gene from the strain *Brevibacillus brevis* encoding an acetolactate decarboxylases enzyme (ALDC) was produced in *B. subtilis* using an integrated expression cassette consisting of the *B. subtilis aprE* promoter, the *B. subtilis aprE* signal peptide sequence, the mature aldB and a BPN' terminator. This cassette was cloned in the head to head orientation with respect to the expression cassette and the *aldB* expression cassette introduced into *B. subtilis* by homologous recombination.

A map of the vector containing the aldB gene (RIHI-aldB) is shown in Figure 1.

The nucleotide mature sequence of the aldB gene in plasmid RIHI-aldB is depicted in SEQ ID NO:4.

gctacaacggctactgtaccagcaccacctgccaagcaggaatccaaacctgcggttgccgctaatccggcaccaaaaaatgtactgtttc
aatactcaacgatcaatgcactcatgcttggacagtttgaaggggacttgactttgaaagacctgaagctgcgaggcgatatggggcttggta
ccatcaatgatctcgatggagagatgattcagatgggtacaaaattctaccagatcgacagcaccggaaaattatcggagctgccagaaagt
gtgaaaactccatttgcggttactacacatttcgagccgaaagaaaaaactacattaaccaatgtgcaagattacaatcaattaacaaaaatgc
ttgaggagaaatttgaaaacaagaacgtcttttatgccgtaaagctgaccggtacttttaagatggtaaaggctagaacagttccaaaacaaa
ccagaccttatccgcagctgactgaagtaaccaaaaaacaatccgagtttgaatttaaaaatgttaagggaaccctgattggcttctatacgcc
aaattatgcagcagccctgaatgttcccggattccatctccacttcatcacagaggataaaacaagtggcggacacgtattaaatctgcaattt
gacaacgcgaatctggaaatttctccgatccatgagtttgatgttcaattgccgcacacagatgattttgcccactctgatctgacacaagttact
actagccaagtacaccaagctgagtcagaaagaaaa

The amino acid sequence of the aldB precursor protein expressed from plasmid RIHI-aldB is depicted in SEQ ID NO:5.

***vrskklwisllfaltliftmafsnmsaqa***ATTATVPAPPAKQESKPAVAANPAPKNVLFQYSTINALML
GQFEGDLTLKDLKLRGDMGLGTINDLDGEMIQMGTKFYQIDSTGKLSELPESVKTPFAV
TTHFEPKEKTTLTNVQDYNQLTKMLEEKFENKNVFYAVKLTGTFKMVKARTVPKQTRP
YPQLTEVTKKQSEFEFKNVKGTLIGFYTPNYAAALNVPGFHLHFITEDKTSGGHVLNLQF
DNANLEISPIHEFDVQLPHTDDFAHSDLTQVTTSQVHQAESERK

The signal peptide sequence is in bold lowercase italics in SEQ ID NO:5.

Small scale culture conditions

To produce aldB, a *B. subtilis* strain transformant containing *aldB* expression cassette was cultured in 15 mL Falcon tubes for 5 hours in TSB (broth) with 300 ppm beta-chloro-D-alanine (CDA), and 300 μL of this pre-culture was added to a 500 mL flask filled with 30 mL of cultivation media (described below) supplemented with 300 ppm CDA and 50μM $Zn^{2+}$. The flasks were incubated for 24, 48 and 72 hours at 33°C with constant rotational mixing at 180 rpm. Cultures were harvested by centrifugation at 14500 rpm for 20 minutes in conical tubes. The culture supernatants were used for protein determination and assays. The cultivation media was an enriched semi-defined media based on MOPs buffer, with urea as major nitrogen source, maltrin as the main carbon source.

Fed-batch fermentation conditions

To produce aldB, a *B. subtilis* strain transformant containing *aldB* expression cassette was cultured in a 250 mL flasks containing 30 mL of complex medium with 300 ppm beta-chloro-D-alanine (CDA). The flask was incubated for 6 hours at 37°C with constant rotational mixing at 180 rpm.

The culture was transferred to a stirred fermentor containing 7 liters of sterilized media components as described in table 1 below. Temperature was controlled to 37°C; pH was controlled to 7,5 using ammonium hydroxide as alkaline titrant; dissolved oxygen was maintained at 40% or higher by maintaining an airflow of 7 liter/min, a constant overpressure of 1 bar and adjusting stirring rate. When initial glucose was exhausted a feeding profile feeding a 60% glucose solution into the fermentor was initiated (initial feeding rate was 20 g/h linearly increasing to 32,8 g/h over 7 hours and kept constant at that rate until fermentation termination).

Total fermentation time was 44 hours.

*Table 1 Media recipe for ALDC fermentation*

| Component | Recipe Conc (g/kg broth) |
|---|---|
| Soy Meal | 50,0 |
| Citric acid | 0,10 |
| Magnesium sulfate heptahydrate | 2,29 |
| Potassium Phosphate, Mono Basic | 5,44 |
| Ferrous sulfate, heptahydrate | 0,029 |
| Manganese Sulfate Mono hydrate | 0,051 |
| Zinc sulphate heptahydrate | 0,001 |
| Glucose mono hydrate | 1,10 |
| Anti foam agent | 3,00 |

**Example 2 - Protein Determination Methods**

Protein Determination by Stain Free Imager Criterion

Protein was quantified by SDS-PAGE gel and densitometry using Gel Doc™ EZ imaging system. Reagents used in the assay: Concentrated (2x) Laemmli Sample Buffer (Bio-Rad, Catalogue #161-0737); 26-well XT 4-12% Bis-Tris Gel ( Bio-Rad, Catalogue #345-0125); protein markers "Precision Plus Protein Standards" (Bio-Rad, Catalogue #161- 0363); protein standard BSA (Thermo Scientific, Catalogue #23208) and SimplyBlue Safestain (Invitrogen, Catalogue #LC 6060. The assay was carried out as follow: In a 96well-PCR plate 50μL diluted enzyme sample were mixed with 50 μL sample buffer containing 2.7 mg DTT. The plate was sealed by Microseal 'B' Film from Bio-Rad and was placed into PCR machine to be heated to 70°C for 10 minutes. After that the chamber was filled by running buffer, gel cassette was set. Then 10 μL of each sample and standard (0.125-1.00 mg/mL BSA) was loaded on the gel and 5 μL of the markers were loaded. After that the electrophoresis was run at 200 V for 45 min. Following electrophoresis the gel was rinsed 3 times 5 min in water, then stained in Safestain overnight and finally destained in water. Then the gel was transferred to Imager. Image Lab software was used for calculation of intensity of each band. By knowing the protein amount of the standard sample, the calibration curve can be made. The amount of sample can be determined by the band intensity and calibration curve. The protein quantification method was employed to prepare samples of aldB acetolactate decarboxylases enzyme used for assays shown in subsequent Examples.

**Example 3 - Activity Assay Method**

Spectrophotometric assay of α-acetolactate decarboxylase

α-Acetolactate decarboxylase (ALDC) catalyses the decarboxylation of α-acetolactate to acetoin. The reaction product acetoin can be quantified colourimetrically. Acetoin mixed with α-naphtol and creatine forms a characteristic red color absorbing at OD522 nm. ALDC activity was calculated based on $OD_{522\ nm}$ and an acetoin calibration curve. The assay was carried out as follows: 20 mM acetolactate substrate was prepared by mixing 100 μL ethyl-2-acetoxy-2-methylacetoacetate (Sigma, Catalogue# 220396) with 3.6 mL 0.5 M NaOH at 10°C for 10 min. 20 mL 50 mM MES pH 6.0 was added, pH was adjusted to pH 6.0 and volume adjusted to 25 mL with 50 mM MES pH 6.0. 80 μL 20 mM acetolactate substrate was mixed with 20 μL enzyme sample diluted in 50 mM MES, pH 6.0, 0.6 M NaCl, 0.05% BRIJ 35 and 0.01% BSA. The substrate/enzyme mixture was incubated at 30°C for 10 min. Then 16 μL substrate/enzyme mixture was transferred to 200 μL 1 M NaOH, 1.0% α-naphtol (Sigma, Catalogue# 33420) and 0.1% creatine (Sigma, Catalogue# C3630). The substrate/enzyme/color reagent mixture was incubated at 30°C for 20 min and then $OD_{522\ nm}$ was read. One unit of ALDC activity is defined as the amount of enzyme which produces 1 μ mole acetoin per minute under the conditions of the assay.

**Example 4 - Zinc influence on activity of ALDC**

The presence of $Zn^{2+}$ during shake flask fermentation

$ZnSO_4$ was added to the cultivation media for small scale fermentations as described in Example 1 at concentrations ranging from 0 mM to 800 μM. aldB. *B. subtilis* transformant containing *aldB* expression cassette was cultured in 15 mL Falcon tubes for 5 hours in TSB (broth) with 300 ppm beta-chloro-D-alanine (CDA), and 300 μL of this preculture was added to a each 500 mL flask filled with 30 mL of cultivation media supplemented with 300 ppm CDA and containing 0, 5, 25, 50, 100, 200, 400 and 800 μM $Zn^{2+}$. The flasks were incubated for 24 and 48 hours at 33°C with constant rotational mixing at 180 rpm. Cultures were harvested by centrifugation at 14500 rpm for 20 minutes in conical tubes. The culture supernatants were used for protein determination and ALDC activity assays (Table 2 and 3). It is clearly seen that increasing the concentration of $Zn^{2+}$ in the fermentation media increased the total ALDC activity and specific activity of expressed aldB enzyme.

*Table 2 ALDC activity of aldB fermentation samples with varying levels of $Zn^{2+}$ after 24 and 48 hours of fermentation*

| | | **ALDC activity** | |
|---|---|---|---|
| | **$ZnSO_4$ in sample** | **24 hours of fermentation** | **48 hours of fermentation** |
| | **μM** | **U/mL** | **U/mL** |
| **aldB** | 0 | 298.6 | 235.3 |
| | 5 | 371.9 | 354.8 |
| | 25 | 533.9 | 808.0 |
| | 50 | 606.4 | 965.3 |
| | 100 | 633.4 | 668.8 |
| | 200 | 617.6 | 629.5 |
| | 400 | 691.1 | 488.5 |
| | 800 | 428.1 | 474.0 |

*Table 3 ALDC activity, aldB protein and specific activity of aldB fermentation samples with varying levels of $Zn^{2+}$ after 48 hours of fermentation*

| | **$ZnSO_4$ in sample** | **ALDC activity** | **AldB protein** | **Specific activity** |
|---|---|---|---|---|
| | **μM** | **U/mL** | **mg/mL** | **U/mg** |
| **aldB** | 0 | 235.3 | 0.626 | 375.9 |
| | 5 | 354.8 | 0.736 | 482.1 |
| | 25 | 808.0 | 1.437 | 562.3 |
| | 50 | 965.3 | 1.573 | 609.9 |
| | 100 | 668.8 | 0.957 | 698.9 |

**Example 5 - Modulation of the specific activity of aldB samples**

Zinc dose response of ALDC at 50°C

$ZnSO_4$ was added to ALDC fermentation sample to produce concentrations ranging from 0 mM to 20 mM. The samples were kept at 50°C for 60 min and then activity was measured as described in Example 3. Relative to a sample with no zinc added samples with >0.5 mM zinc had activity >200% higher. Figure 2 shows results.

**Example 6 - Modulation of the specific activity of aldB ferment**

AldB activity was produced as described in (Example 1) and activity determined as described in (Example 3). An AldB ferment sample was diluted in 50 mM MES pH 6.0, 0.6 M NaCl, 0,05% Brij, 0.01% BSA (bovine serum albumin) to proximately 1000 U/mL and then incubated with varying concentration of $ZnSO_4$ in the following steps: 0, 0.25, 0.5, 1, 2, 5, 7.5, 10, 20, 40, 60, 80, 100, 120mM. All samples were incubated at 4°C for 68 hours, then centrifuged and supernatant analyzed for activity and protein by Criterion SDS-PAGE analysis. The results of the SDS-PAGE analysis is shown in Figure 3 and the determined activity, protein concentration and specific activity are given in table 4. It can be seen that the ALDC activity increases with increasing concentration of $ZnSO_4$ in the sample until approximately 5-10 mM from where it is more or less constant with the higher concentrations of $ZnSO_4$. The concentration of aldB protein in the sample was determined to be more or less constant in all samples indicating an apparent increase in the specific activity from 968 U/mg in the sample without $ZnSO_4$ up to 2200-2400 U/mg in samples with 5mM $ZnSO_4$ or more after incubation.

*Table 4 ALDC activity, aldB protein concentration and specific activity of aldB sample incubated with $ZnSO_4$ at 4 °C for 68 hours.*

| | **$ZnSO_4$ in sample** | **ALDC activity** | **AldB protein** | **Specitic activity** |
|---|---|---|---|---|
| | **mM** | **U/mL** | **mg/mL** | **U/m** |
| **aldB** | 0 | 556 | 0.57 | 968 |
| | 0.25 | 875 | 0.63 | 1387 |
| | 0.5 | 949 | 0.63 | 1512 |
| | 1 | 1058 | 0.59 | 1792 |
| | 2 | 1271 | 0.60 | 2124 |
| | 5 | 1411 | 0.60 | 2354 |
| | 7.5 | 1485 | 0.64 | 2311 |
| | 10 | 1520 | 0.64 | 2368 |
| | 20 | 1492 | 0.64 | 2344 |
| | 40 | 1499 | 0.67 | 2239 |
| | 60 | 1495 | 0.64 | 2345 |
| | 80 | 1500 | 0.65 | 2324 |
| | 100 | 1607 | 0.64 | 2520 |
| | 120 | 1520 | 0.69 | 2209 |

**Example 7 - Purification of aldB**

Production of aldB in *B. subtilis* was carried out as described in example (1). Fermentation media was clarified by centrifugation (4000 rpm at 15 min.) and filtration (VacuCap 90, 0.2 $\mu$m). The sample was desalted on PD10 column, prepared as described by the manufacturer and equilibrated with 20 mM Tris/HCl, pH 8.0. The eluate was kept on ice afterwards before further purified by anion exchange chromatography on a Source 15Q XK26/15. The column was equipped on an ÄKTA explorer system used for protein purification and according to the method described by the manufacturer (GE healthcare, USA - Cat. No. 18-1112-41).

The column was prepared as described by the manufacturer and equilibrated with 20 mM Tris/HCl, pH 8.0 (buffer A). The desalted sample was applied to the column at a flow rate of 5 mL/min and the column was washed with buffer A. The bound proteins were eluted with a linear gradient of 0-0.30 M NaCl in 20 mM Tris/HCl, pH 8.0 (7 mL/min, 40 min). During the entire run, fractions of approx. 10-20 mL were collected.

Fractions from purification were analysed by SDS-PAGE using Xcell Mini-cell and according to the method described by the manufacturer (Invitrogen, USA - Cat. No. EI0001) using: Nu-PAGE gel (Invitrogen, USA - Cat. No. NP0321), MES running buffer (Invitrogen, USA - Cat. No. NP0002), See Blue standard marker (Invitrogen, USA - Cat. No. LC5925) and Stained with Coomasie Brilliant-Blue.

AldB was observed to bind at the Source 15Q column and was eluted with approximately 30 mM NaCl. AldB fractions were collected, concentrated (on a 10 kDa UF Vivaspin) and desalted in 25 mM HEPES buffer containing 150 mM NaCl, pH 8.0. The estimated purity of aldB was above 95% and the result of SDS-page analysis of the purified aldB is shown in figure 4.

**Example 8 - Modulation of the specific activity of purified aldB**

The purified aldB protein was stripped of divalent ions by incubation of aldB (approximately 2 mg/mL) with 20mM EDTA in 0.2x assay buffer (50 mM MES pH 6.0, 0.6 M NaCl, 0,05% Brij, 0.01% BSA) at 37°C overnight. The EDTA treated material was desalted on a PD10 column prepared as described by the manufacturer and equilibrated with 50 mM MES pH 6.0, 0.6 M NaCl, 0,05% Brij, 0.01% BSA. ALDC activity and the concentration of AldB protein in the sample before and after EDTA treatment and the desalting column were determined as described in example (2 and 3) and the results are shown in table 5.

*Table 5 ALDC activity, aldB protein concentration and specific activity of purified aldB before and after the EDTA-desalting procedure.*

| | ALDC Activity | AldB protein concentration | Specific Activity |
|---|---|---|---|
| | U/mL | mg/mL | U/mg |
| AldB purified | 5187 | 5.6 | 928 |
| AldB purified - EDTA treated and desalted | 9 | 1.0 | 9 |

50μL of the EDTA treated and desalted aldB sample was mixed with 450μL of 0.2x assay buffer (50 mM MES pH 6.0, 0.6 M NaCl, 0,05% Brij, 0.01% BSA) with varying $ZnSO_4$ in the step: 0, 0.05, 0.1, 0.5, 1, 2, 5, 10 and 20mM respectively. Samples were incubated at 37°C overnight. The samples were centrifuged at 13000 rpm for 10 min and ALDC activity and the concentration of AldB protein were determined in the supernatant. The results are shown in table 6. It can be seen that the ALDC activity increases with increasing concentration of $ZnSO_4$ during incubation. The concentration of aldB protein in the sample was determined to be constant in all samples indicating an increase in the specific activity from 2 U/mg of aldB in the sample without $ZnSO_4$ up to 1800 U/mg aldB in samples with 5mM $ZnSO_4$ or more after incubation.

*Table 6 ALDC activity, aldB protein concentration and specific activity of purified aldB before and after incubation with $ZnSO_4$.*

| Concentration of $ZnSO_4$ in 0,2X assay buffer | Final concentration of $ZnSO_4$ | ALDC Activity | aldB protein concentration | Specific activity |
|---|---|---|---|---|
| mM | mM | U/mL | mg/mL | U/mg |
| 0 | 0 | 0,1 | 0,092 | 2 |
| 0,05 | 0,045 | 4,4 | 0,092 | 48 |
| 0,1 | 0,09 | 26,1 | 0,092 | 283 |
| 0,5 | 0,45 | 147,1 | 0,092 | 1599 |
| 1 | 0,9 | 157,7 | 0,092 | 1714 |
| 2 | 1,8 | 157,9 | 0,092 | 1716 |
| 5 | 4,5 | 166,4 | 0,092 | 1809 |
| 10 | 9 | 174,6 | 0,092 | 1898 |
| 20 | 18 | 173,5 | 0,092 | 1886 |

In addition, 20μL of the EDTA treated and desalted aldB sample was mixed with 180μL of 50 mM MES pH 6.0 supplemented with either 10mM $ZnSO_4$, $MnSO_4$ or $CoCl_2$ respectively. Samples were incubated at 37°C and assayed after 45 minutes, 1 day, 2 days and 3 days. Results are shown in table 7. The activity increased for incubation with all three divalent ions in the order $Zn^{2+}$, $Mn^{2+}$ and $Co^{2+}$ compared to the control (addition of $H_2O$). After 45 min the activity was shown to decrease over time. The addition of $Zn^{2+}$, $Mn^{2+}$ and $Co^{2+}$ all resulted in an increased stability (activity) after 3 days compared to the control with addition of $H_2O$. The residual activity measured after 3 days relative to the start were 76,9%, 73,5% and 70,7% upon addition of $Zn^{2+}$, $Mn^{2+}$ and $Co^{2+}$ compared to 56,0% obtained with $H_2O$, as shown in table 7.

*Table 7 ALDC activity after incubation at pH 6.0 with $MnSO_4$, $CoCl_2$ or $ZnSO_4$ at various times.*

| | Activity U/mL | | | |
|---|---|---|---|---|
| | $H_2O$ | $Mn^{2+}$ | $Co^{2+}$ | $Zn^{2+}$ |
| 45min | 21.8 | 217.3 | 101.8 | 319.7 |
| 1 day | 18.9 | 219.6 | 90.5 | 288.5 |
| 2 days | 22.5 | 216.2 | 95.2 | 295.4 |
| 3 days | 12.2 | 153.6 | 74.8 | 245.7 |

**Example 9 - Reduction in diacetyl and 2,3-pentanedione during beer fermentation by use of aldB with different specific ALDC activity**

The objective of this analysis was to test different formulation variants of aldB (acetolactate decarboxylase) ability to reduce development of diacetyl and 2,3-pentanedione (Vicinal di-ketones, VDK) during a 7 days fermentation at 14°C.

Pure malt brew analysis

1100 g Munton's Light Malt Extract (Batch XB 35189, expiry date 24.05.2014) extract was dissolved in 3000 mL warm tapwater (45°C). This slurry was stirred for about 10 min until the liquid was homogeneous and the pH was adjusted to 5.2 with 2.5 M sulphuric acid. To the slurry was added 10 pellets of Bitter hops from Hopfenveredlung, St. Johann: Alpha content of 16,0 % (EBC 7.7 0 specific HPLC analysis, 01.10.2013), then split in 500mL blue-cap bottles and boiled for 1 hour to ensure protein precipitation and avoid potential microbial contamination. The filtered malt extract (wort) was sampled for specific gravity and Free Amino Nitrogen (FAN) determination. The final wort had an initial Specific Gravity of 1048 (i.e. 12 °Plato). 200g of the filtered wort were added to a 500 mL conical flask (Fermenting Vessel; FV), and then cooled to 13°C. Each conical flask was dosed with 0.5% W34/70 (Weihenstephan) freshly produced yeast (1.0g yeast per 200 g wort). The enzymes were dosed on similar ALDC activity (0.03 U/mL wort, 8 ALDC Units per 200 g wort). The control fermentation vessel with no enzyme received an amount of deionized water corresponding to the amount of enzyme sample.

The wort samples were fermented in 500 mL conical flasks under standardised laboratory test conditions at 14°C with gentle agitation at 150 rpm in an orbital incubator. When weight loss was less than 0.25g over 24 hours, fermentation temperature was decreased to 7°C. Fermentation was stopped after 7 days in total. 10 mL samples were taken out for diacetyl and 2,3-pentanedione analysis two times a day, preferably with 11 to 14 hours in between; at the end of fermentation only 1 sample per day was taken. Yeast was allowed to settle before take-out and each sample was cooled at 10°C for 10 minutes and then centrifuged at 4000rpm for 10 minutes at 8°C to sediment any residual yeast. The supernatant was separated from the yeast sediment and samples for GC analysis were added 0.5 g NaCl per mL of sample. This slurry was transferred to a headspace vial and heat-treated at 65°C for 30 minutes before analysis for diacetyl and 2,3-pentanedione were carried out by gas chromatography with mass spectrometric detection (GCMS).

Analyses were carried out at an Agilent 6890N/5973N GC with CombiPAL headspace autosampler and MSChemStation acquisition and analysis software. The samples were equilibrated at 70°C for 10 minutes before 500μl of the gas phase above the sample was injected onto a J&W 122-0763 DB-1701column (60m × 0.25mmID × 1 μm). The injection temperature was 260°C and the system was operated with a constant helium flow of 2 mL/min. The oven temperature was: 50°C (2 min), 160°C (20°C/min), 220°C (40°C/min), hold 2 min. MS detection were made with 500 μL at a split ratio of 5:1at selected ions. All sample were run in duplicates and standards were made using tap water with the addition of diacetyl or 2,3-pentanedione.

The concentration of a compound is calculated as

$$\text{Compound (mg/L)} = \text{RF x } \frac{\text{Area}}{1000 \text{ x } W_s}$$

where,

RF is the response factor of acetic acid
Area is the GC-area of acetic acid
$W_s$ is the amount of sample used (in mL)

The limit of diacetyl quantification was determined to 0.016 mg/L and the limit of 2,3-pentanedione quantification was determined to 0.012 mg/L.

To check that addition of ALDC enzymes did not influence the Real Degree of Fermentation (RDF) and the produced alcohol by volume: RDF was measured using an Anton Paar (DMA 5000) following Standard Instruction Brewing, 23.8580-B28 and alcohol by Standard Instruction Brewing, 23.8580-B28.

Real degree of fermentation (RDF) value may be calculated according to the equation below:

$$RDF(\%) = \left(1 - \frac{RE}{^{o}P_{initial}}\right) \times 100$$

Where: RE = real extract = (0.1808 × $°P_{initial}$) + (0.8192 × $°P_{final}$), $°P_{initial}$ is the specific gravity of the standardised worts before fermentation and $°P_{final}$ is the specific gravity of the fermented worts expressed in degree Plato.

In the present context, Real degree of fermentation (RDF) was determined from the specific gravity and alcohol concentration.

Specific gravity and alcohol concentration was determined on the fermented samples using a Beer Alcolyzer Plus and a DMA 5000 Density meter (both from Anton Paar, Gratz, Austria). Based on these measurements, the real degree of fermentation (RDF) value was calculated according to the equation below:

$$RDF(\%) = \frac{OE - E(r)}{OE} \times 100$$

Where: E(r) is the real extract in degree Plato (°P) and OE is the original extract in °P.

The ability to reduce development of diacetyl and 2,3-pentanedione (Vicinal di-ketones, VDK) during a 7 days fermentation at 14°C was studied by addition of aldB with different specific ALDC activity (obtained by varying the $ZnSO_4$ concentration in the sample) is given in table 8. The aldB variant constitute: A) a crude preparation of aldB, B) a crude preparation of aldB including 20mM $ZnSO_4$ added to the clarified broth and C) a crude preparation of aldB including 50μM $ZnSO_4$ added to the fermentation media and 20mM $ZnSO_4$ into the clarified broth.

*Table 8 ALDC activity, aldB protein concentration and specific activity of aldB sample A, B and* C.

| | **ALDC activity** | **aldB protein** | **Specific ALDC activity** |
|---|---|---|---|
| | **U/mL** | **mg/mL** | **U/mg** |
| **aldB variant A** | 426 | 0,463 | 919 |
| **aldB variant B** | 511 | 0,463 | 1103 |
| **aldB variant C** | 719 | 0,463 | 1552 |

The enzymes variants were dosed on similar ALDC activity 0.03 U/mL wort resulting in a different concentration of aldB protein in the wort, as seen in table 9. The calculated aldB protein concentration in the wort was 33.4, 28.0 and 19.9 μg/L wort with addition of variant A, B and C respectively.

*Table 9 ALDC activity and aldB protein in wort with addition of aldB variant A, B and C.*

| | **ALDC activity** | **Amount sample for pre-dilution** | **Volume pre-dilution** | **Activity in wort** | **AldB protein in wort** |
|---|---|---|---|---|---|
| | **U/mL** | **g** | **mL** | **U/mL** | **μg/l** |
| **AldB variant A** | 426 | 0,721 | 50 | 0,03 | 33,4 |

(continued)

| | ALDC activity | Amount sample for pre-dilution | Volume pre-dilution | Activity in wort | AldB protein in wort |
|---|---|---|---|---|---|
| | U/mL | g | mL | U/mL | μg/l |
| AldB variant B | 511 | 0,604 | 50 | 0,03 | 28,0 |
| AldB variant C | 719 | 0,430 | 50 | 0,03 | 19,9 |

The fermentations were conducted and VDK development analysed as described above. Fermentations with enzyme were compared to a control without any enzyme added. The development of VDK is shown in figure 5.

All three aldB variants reduced the VDK development during fermentation compared to control. The data suggest that the three variants performed very similar, however with less increase in VDK during the fermentation period from 20 to 40 hours for variant C compared to A and B, but this is close to the relative standard deviation of the analysis (RSD of 7.5%). The relative reduction in VDK after 40 hours of fermentation was 63.1 %, 58.8 % and 63.2 % for variant A, B and C respectively. In addition, the fermentation time required to reach threshold level of 0.1 mg/ml VDK or lower, was observed to be approximately 112 hours for all three variants. Thus, the higher specific activity of variant C compared to A or B may enable similar VDK reduction with less aldB protein.

**Example 10 - Reduction in diacetyl and 2,3-pentanedione by aldB during beer fermentation with various concentration of zinc in the wort**

The objective of this analysis was to test reduction of diacetyl and 2,3-pentanedione (Vicinal di-ketones, VDK) by aldB during a 4 days fermentation at 14°C in presence of various level of $ZnSO_4$ in the wort.

The brew analysis setup was as described in example 9. A stock solution of $ZnSO_4$ was made in cooled wort (0.686 g in 1000mL wort) and applied to the wort samples (prepared as described in example 9) before fermentation resulting in the following $Zn^{2+}$ concentrations 0, 0.1, 0.5, 0.75, 1, 2.5, 5.0 and 20.0 ppm respectively. ALDC enzyme and yeast were subsequently added to the samples. An ALDC enzymes preparation were dosed similarly to all samples (0.04 U/mL wort, corresponding to 8 ALDC units per 200 g of wort). Each conical fermentation flask was dosed with 0.5% W34/70 (Weihenstephan) freshly produced yeast (1.0g yeast per 200 g of wort) and analyses were carried out as described in example 9. Fermentations with enzyme were compared to a control without any enzyme added. The development of VDK is shown in figure 6.

All aldB treated samples provided less increase in the VDK development during fermentation compared to control. Increasing the concentration of $Zn^{2+}$ in the samples resulted in less and less increase in VDK generation. Especially increasing the concentration of $Zn^{2+}$ to 0.5ppm or more greatly affected the enzymatic activity to avoid generation of VDK. Supplementing the wort with 1 ppm $Zn^{2+}$ or more ensured a VDK level below the flavor threshold of 0.1mg/L throughout the whole fermentation period.

## Claims

1. A method for increasing the activity and/or stability of an ALDC enzyme of EC 4.1.1.5 wherein said method comprises the step of adding zinc to a fermentation media and/or maturation media comprising an ALDC producing host cell during a beer and/or wine and/or cider and/or perry and/or sake fermentation:

   (i) at a concentration of 1 μM to 300 μM, preferably 6 μM to 300 μM; or
   (ii) at a concentration of 1 μM to 50 μM; or
   (iii) at a concentration of 1 μM to 25 μM, preferably 6 μM to 25 μM; or
   (iv) as a composition comprising ALDC and zinc, wherein:

      (a) said zinc is present in said composition at a concentration of 1 mM to 5 mM; or
      (b) the molar ratio of zinc to ALDC enzyme in the composition is higher than 1; or 2:1 or higher; or 10:1 or higher; or 20:1 or higher; or 30:1 or higher; or 60:1 or higher.

2. The method of claim 1, wherein said ALDC enzyme and said zinc are added during a fermentation process or a maturation process.

3. The method of claim 1 or claim 2, wherein said ALDC enzyme is added at a concentration of 0.5 g to 10 g per hectoliter of beer and/or wine and/or cider and/or perry and/or sake ferment.

4. The method of any preceding claim, wherein said ALDC enzyme is added at a concentration of 1 g to 5 g per hectoliter of beer and/or wine and/or cider and/or perry and/or sake ferment.

**5.** The method of any preceding claim, wherein the activity of said ALDC enzyme is in the range of 1000 to 2500 Units per mg of protein.

**6.** The method of any preceding claim, wherein said ALDC enzyme is from *Lactobacillus casei*, *Brevibacterium acetylicum*, *Lactococcus lactis, Leuconostoc lactis, Enterobacter aerogenes, Bacillus subtilis, Bacillus brevis, Lactococcus lactis DX,* or *Bacillus licheniformis,* preferably wherein the ALDC enzyme is from *Bacillus brevis* or *Bacillus licheniformis.*

**7.** The method of any preceding claim, wherein said ALDC enzyme has an amino acid sequence having at least 80% identity with any one selected from SEQ ID NO: 2, SEQ ID NO: 3, SEQ ID NO: 5, SEQ ID NO: 7 and SEQ ID NO: 8 or any functional fragment thereof.

**8.** A beer and/or wine and/or cider and/or perry and/or sake fermentation media and/or maturation media comprising a composition comprising an ALDC enzyme of EC 4.1.1.5 and zinc wherein said composition comprises zinc at a concentration of 1 μM to 200 mM, preferably wherein:

(i) zinc is present in the composition at a concentration of 1 μM to 300 μM, preferably 6 μM to 300 μM; or
(ii) zinc is present in the composition at a concentration of 1 μM to 50 μM, preferably 6 μM to 50 μM, or 6 μM to 25 μM; or
(iii) the zinc and the ALDC enzyme are added in a composition, wherein zinc is present in the composition at a concentration of 1 mM to 20 mM, preferably 1 mM to 5 mM; or
(iv) the zinc and the ALDC enzyme are added in a composition, where the molar ratio of zinc to ALDC enzyme in the composition is higher than 1; or 2:1 or higher; or 10:1 or higher; or 20:1 or higher; or 30:1 or higher; or 60:1 or higher.

**9.** The beer and/or wine and/or cider and/or perry and/or sake fermentation media and/or maturation media of claim 8, wherein: the activity of said ALDC enzyme is in the range of 1000 to 2500 Units per mg of protein; and/or said media further comprises at least one additional enzyme or enzyme derivative selected from the group consisting of acetolactate reductoisomerases, acetolactate isomerases, amylase, glucoamylase, hemicellulase, cellulase, glucanase, pullulanase, isoamylase, endo-glucanase and related beta-glucan hydrolytic accessory enzymes, xylanase, xylanase accessory enzymes (for example, arabinofuranosidase, ferulic acid esterase, and xylan acetyl esterase) and protease.

## Patentansprüche

**1.** Verfahren zum Erhöhen der Aktivität und/oder der Stabilität eines ALDC-Enzyms von EC 4.1.1.5, wobei das Verfahren den Schritt des Zugebens von Zink zu einem Fermentationsmedium und/oder Reifungsmedium während einer Fermentation von Bier und/oder Wein und/oder Cider und/oder Perry und/oder Sake umfasst, welches eine ALDCproduzierende Wirtszelle umfasst:

(i) bei einer Konzentration von 1 μM bis 300 μM, vorzugsweise 6 μM bis 300 μM; oder
(ii) bei einer Konzentration von 1 μM bis 50 μM; oder
(iii) bei einer Konzentration von 1 μM bis 25 μM, vorzugsweise 6 μM bis 25 μM; oder
(iv) als eine Zusammensetzung, die ALDC und Zink umfasst, wobei:

(a) das Zink in der Zusammensetzung in einer Konzentration von 1 mM bis 5 mM vorliegt; oder
(b) das Molverhältnis von Zink zu ALDC-Enzym in der Zusammensetzung höher als 1 ist; oder mindestens 2:1; oder mindestens 10:1; oder mindestens 20:1; oder mindestens 30:1; oder mindestens 60:1 beträgt.

**2.** Verfahren nach Anspruch 1, wobei das ALDC-Enzym und das Zink während eines Fermentationsprozesses oder eines Reifungsprozesses zugegeben werden.

**3.** Verfahren nach Anspruch 1 oder 2, wobei das ALDC-Enzym in einer Konzentration von 0,5 g bis 10 g pro Hektoliter Bier- und/oder Wein- und/oder Cider- und/oder Perry- und/oder Sake-Ferment zugegeben wird.

**4.** Verfahren nach einem der vorhergehenden Ansprüche, wobei das ALDC-Enzym in einer Konzentration von 1 g bis 5 g pro Hektoliter Bier- und/oder Wein- und/oder Cider- und/oder Perry- und/oder Sake-Ferment zugegeben wird.

5. Verfahren nach einem der vorhergehenden Ansprüche, wobei die Aktivität des ALDC-Enzyms im Bereich von 1000 bis 2500 Einheiten pro mg Protein liegt.

6. Verfahren nach einem der vorhergehenden Ansprüche, wobei das ALDC-Enzym von *Lactobacillus casei, Brevibacterium acetylicum, Lactococcus lactis, Leuconostoc lactis, Enterobacter aerogenes, Bacillus subtilis, Bacillus brevis, Lactococcus lactis DX* oder *Bacillus licheniformis* stammt, vorzugsweise wobei das ALDC-Enzym von *Bacillus brevis* oder *Bacillus licheniformis* stammt.

7. Verfahren nach einem der vorhergehenden Ansprüche, wobei das ALDC-Enzym eine Aminosäuresequenz mit einer Identität von mindestens 80 % zu einer beliebigen von SEQ ID NO: 2, SEQ ID NO: 3, SEQ ID NO: 5, SEQ ID NO: 7 und SEQ ID NO: 8 oder zu einem beliebigen funktionellen Fragment davon aufweist.

8. Fermentationsmedium und/oder Reifungsmedium für Bier und/oder Wein und/oder Cider und/oder Perry und/oder Sake, umfassend eine Zusammensetzung, welche ein ALDC-Enzym von EC 4.1.1.5 und Zink umfasst, wobei die Zusammensetzung Zink in einer Konzentration von 1 μM bis 200 mM umfasst, vorzugsweise wobei:

   (i) Zink in der Zusammensetzung in einer Konzentration von 1 μM bis 300 μM vorliegt, vorzugsweise von 6 μM bis 300 μM; oder
   (ii) Zink in der Zusammensetzung in einer Konzentration von 1 μM bis 50 μM vorliegt, vorzugsweise von 6 μM bis 50 μM oder von 6 μM bis 25 μM; oder
   (iii) das Zink und das ALDC-Enzym in einer Zusammensetzung zugegeben werden, wobei Zink in der Zusammensetzung in einer Konzentration von 1 mM bis 20 mM vorliegt, vorzugsweise von 1 mM bis 5 mM; oder
   (iv) das Zink und das ALDC-Enzym in einer Zusammensetzung zugegeben werden, wobei das Molverhältnis von Zink zu ALDC-Enzym in der Zusammensetzung höher als 1 ist; oder mindestens 2:1; oder mindestens 10:1; oder mindestens 20:1; oder mindestens 30:1; oder mindestens 60:1 beträgt.

9. Fermentationsmedium und/oder Reifungsmedium für Bier und/oder Wein und/oder Cider und/oder Perry und/oder Sake nach Anspruch 8, wobei: die Aktivität des ALDC-Enzyms im Bereich von 1000 bis 2500 Einheiten pro mg Protein liegt; und/oder das Medium ferner mindestens ein zusätzliches Enzym oder Enzymderivat umfasst, ausgewählt aus der Gruppe, bestehend aus Acetolactatreduktoisomerasen, Acetolactatisomerasen, Amylase, Glucoamylase, Hemicellulase, Cellulase, Glucanase, Pullulanase, Isoamylase, Endoglucanase und verwandte hydrolytische Beta-Glucan-Hilfsenzyme, Xylanase, Xylanase-Hilfsenzyme (z. B. Arabinofuranosidase, Ferulasäureesterase und Xylanacetylesterase) und Protease.

## Revendications

1. Procédé pour l'augmentation de l'activité et/ou de la stabilité d'une enzyme ALDC de EC 4.1.1.5, ledit procédé comprenant l'étape d'ajout de zinc à un milieu de fermentation et/ou un milieu de maturation comprenant une cellule hôte produisant de l'ALDC pendant une fermentation de bière et/ou de vin et/ou de cidre et/ou de poiré et/ou de saké :

   (i) à une concentration de 1 μM à 300 μM, préférablement de 6 μM à 300 μM ; ou
   (ii) à une concentration de 1 μM à 50 μM ; ou
   (iii) à une concentration de 1 μM à 25 μM, préférablement de 6 μM à 25 μM ; ou
   (iv) en tant qu'une composition comprenant de l'ALDC et du zinc,

      (a) ledit zinc étant présent dans ladite composition à une concentration de 1 mM à 5 mM ; ou
      (b) le rapport molaire de zinc sur enzyme ALDC dans la composition étant supérieur à 1 ; ou 2 : 1 ou plus grand ; ou 10 : 1 ou plus grand ; ou 20 : 1 ou plus grand ; ou 30 : 1 ou plus grand ; ou 60 : 1 ou plus grand.

2. Procédé selon la revendication 1, ladite enzyme ALDC et ledit zinc étant ajoutés pendant un procédé de fermentation ou un procédé de maturation.

3. Procédé selon la revendication 1 ou la revendication 2, ladite enzyme ALDC étant ajoutée à une concentration de 0,5 g à 10 g par hectolitre de ferment de bière et/ou de vin et/ou de cidre et/ou de poiré et/ou de saké.

4. Procédé selon une quelconque revendication précédente, ladite enzyme ALDC étant ajoutée à une concentration de 1 g à 5 g par hectolitre de ferment de bière et/ou de vin et/ou de cidre et/ou de poiré et/ou de saké.

**5.** Procédé selon une quelconque revendication précédente, l'activité de ladite enzyme ALDC étant dans la plage de 1 000 à 2 500 unités par mg de protéine.

**6.** Procédé selon une quelconque revendication précédente, ladite enzyme ALDC provenant de *Lactobacillus casei, Brevibacterium acétylicum, Lactococcus lactis, Leuconostoc lactis, Enterobacter aerogenes, Bacillus subtilis, Bacillus brevis, Lactococcus lactis DX,* ou *Bacillus licheniformis,* préférablement l'enzyme ALDC provenant de *Bacillus brevis* ou *Bacillus licheniformis.*

**7.** Procédé selon une quelconque revendication précédente, ladite enzyme ALDC possédant une séquence d'acides aminés possédant au moins 80 % d'identité avec l'une quelconque choisie parmi la SEQ ID NO: 2, la SEQ ID NO: 3, la SEQ ID NO: 5, la SEQ ID NO: 7 et la SEQ ID NO: 8 ou un quelconque fragment fonctionnel correspondant.

**8.** Milieu de fermentation et/ou milieu de maturation de bière et/ou de vin et/ou de cidre et/ou de poiré et/ou de saké comprenant une composition comprenant une enzyme ALDC de EC 4.1.1.5 et du zinc, ladite composition comprenant du zinc à une concentration de 1 μM à 200 mM, préférablement :

(i) le zinc étant présent dans la composition à une concentration de 1 μM à 300 μM, préférablement de 6 μM à 300 μM ; ou
(ii) le zinc étant présent dans la composition à une concentration de 1 μM à 50 μM, préférablement de 6 μM à 50 μM, ou de 6 μM à 25 μM ; ou
(iii) le zinc et l'enzyme ALDC étant ajoutés dans une composition, le zinc étant présent dans la composition à une concentration de 1 mM à 20 mM, préférablement de 1 mM à 5 mM ; ou
(iv) le zinc et l'enzyme ALDC étant ajoutés dans une composition, où le rapport molaire de zinc sur enzyme ALDC dans la composition est supérieur à 1 ; ou 2 : 1 ou plus grand ; ou 10 : 1 ou plus grand ; ou 20 : 1 ou plus grand ; ou 30 : 1 ou plus grand ; ou 60 : 1 ou plus grand.

**9.** Milieu de fermentation et/ou milieu de maturation de bière et/ou de vin et/ou de cidre et/ou de poiré et/ou de saké selon la revendication 8, l'activité de ladite enzyme ALDC étant dans la plage de 1 000 à 2 500 unités par mg de protéine ; et/ou ledit milieu comprenant en outre au moins un(e) enzyme ou dérivé d'enzyme supplémentaire choisi(e) dans le groupe constitué par des acétolactate réductoisomérases, des acétolactate isomérases, une amylase, une glucoamylase, une hémicellulase, une cellulase, une glucanase, une pullulanase, une isoamylase, une endo-glucanase et des enzymes accessoires hydrolytiques de bêta-glucane liées, une xylanase, des enzymes accessoires xylanase (par exemple, une arabinofuranosidase, une acide férulique estérase, et une acétylxylane estérase) et une protéase.

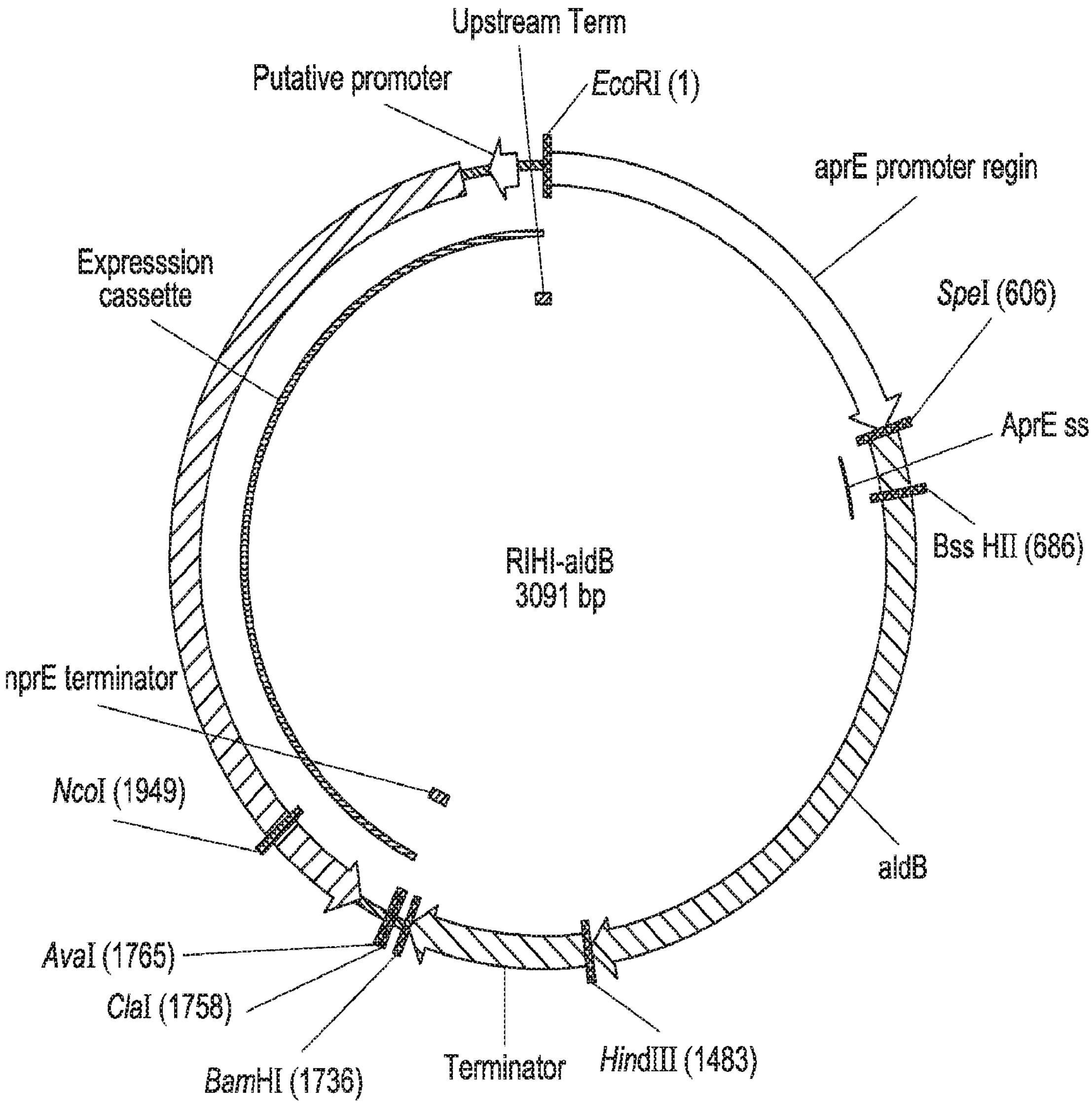
Upstream Term
Putative promoter
EcoRI (1)
aprE promoter regin
Expresssion cassette
SpeI (606)
AprE ss
Bss HII (686)
RIHI-aldB
3091 bp
nprE terminator
NcoI (1949)
aldB
AvaI (1765)
ClaI (1758)
BamHI (1736)
Terminator
HindIII (1483)

*FIG. 1*

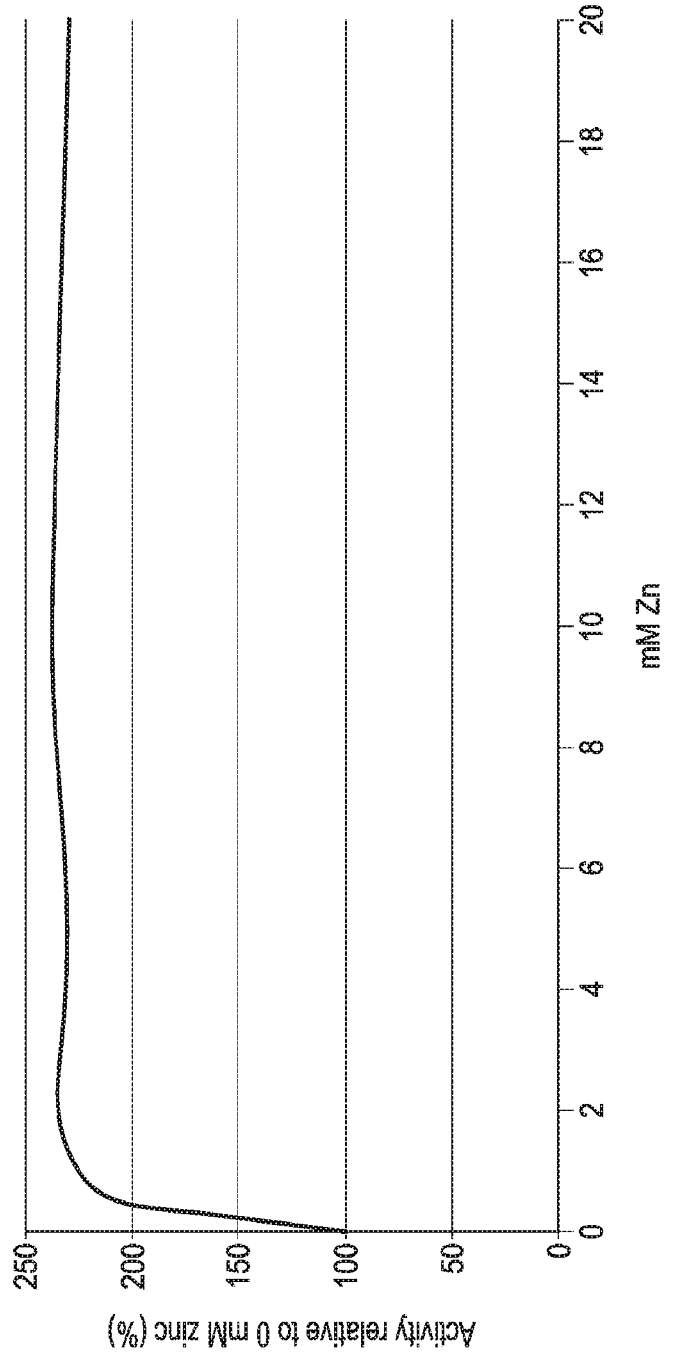
Activity relative to 0 mM zinc (%)
0
50
100
150
200
250
0
2
4
6
8
10
12
14
16
18
20
mM Zn

*FIG. 2*

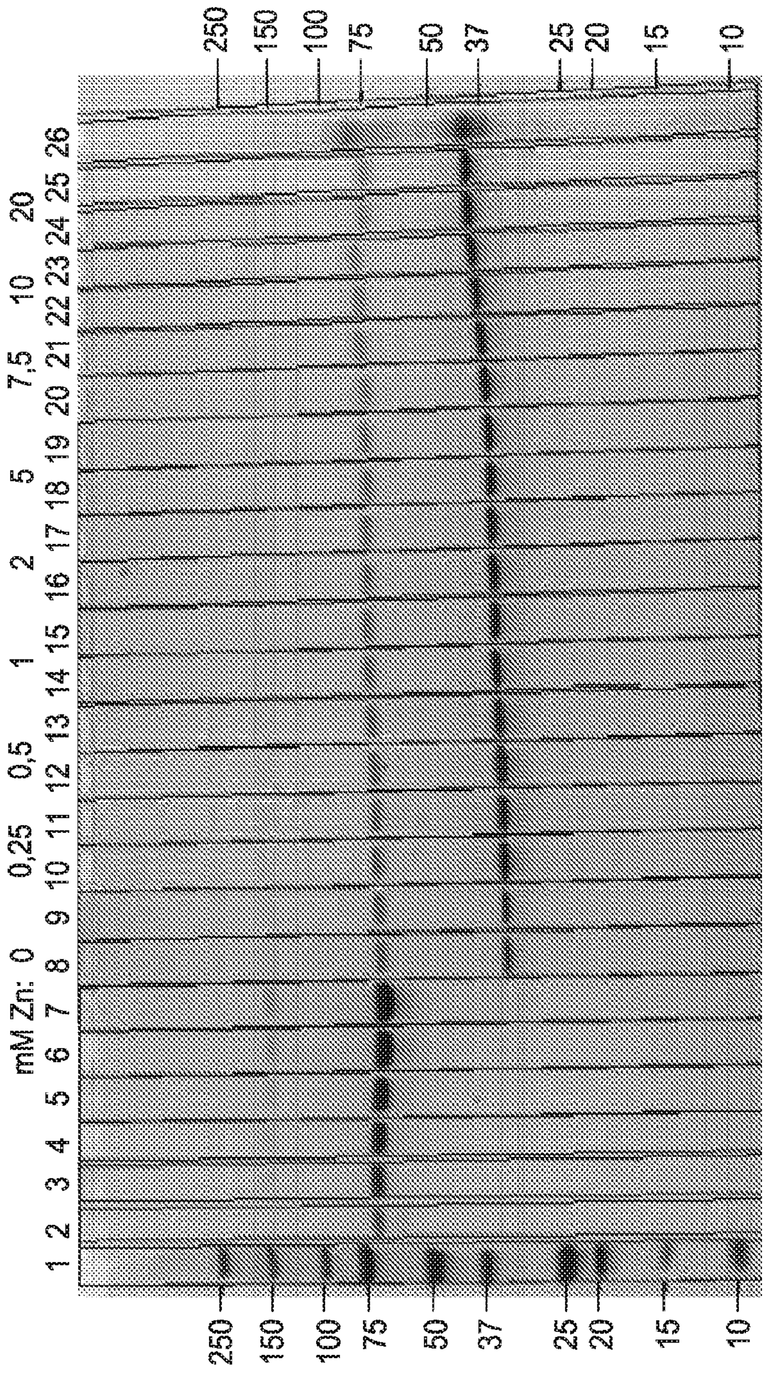

mM Zn: 0
0,25
0,5
1
2
5
7,5
10
20
1 2 3 4 5 6 7 8 9 10 11 12 13 14 15 16 17 18 19 20 21 22 23 24 25 26
Std
250
150
100
75
50
37
25
20
15
10


FIG. 3A

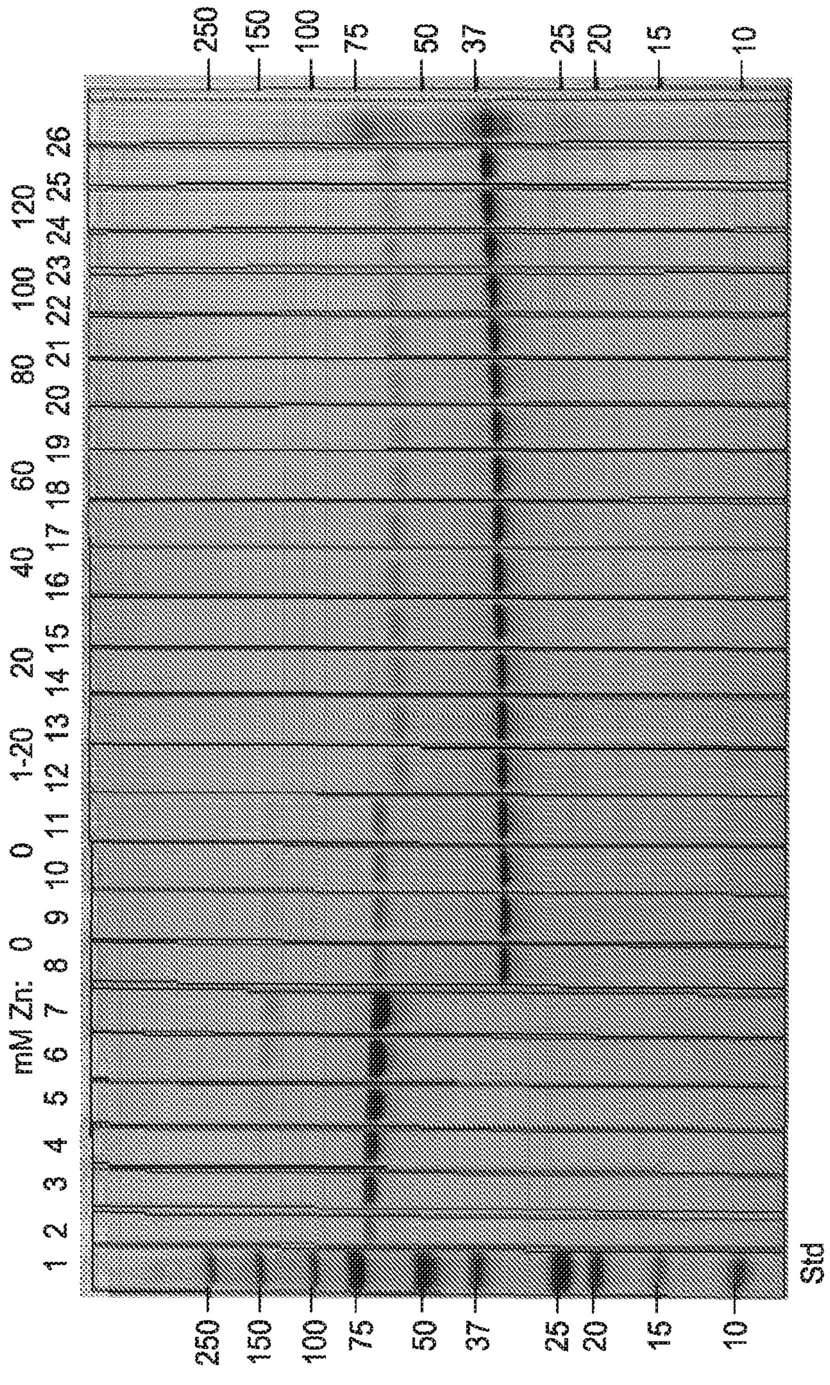
mM Zn: 0 0 1-20 20 40 60 80 100 120
1 2 3 4 5 6 7 8 9 10 11 12 13 14 15 16 17 18 19 20 21 22 23 24 25 26
250
150
100
75
50
37
25
20
15
10
Std

*FIG. 3B*

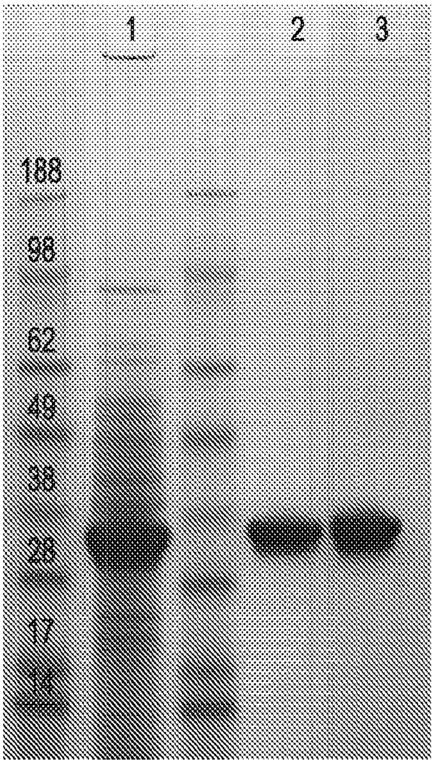


*FIG. 4*

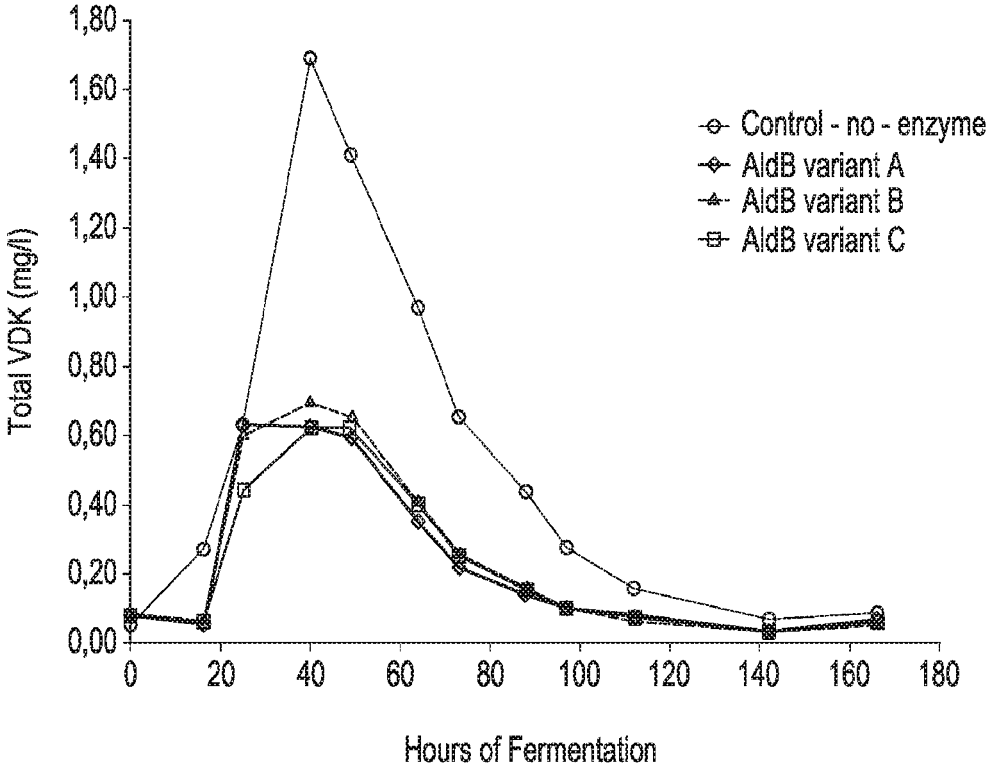
1,80
1,60
1,40
1,20
1,00
0,80
0,60
0,40
0,20
0,00
Total VDK (mg/l)
0
20
40
60
80
100
120
140
160
180
Hours of Fermentation
Control - no - enzyme
AldB variant A
AldB variant B
AldB variant C

*FIG. 5*

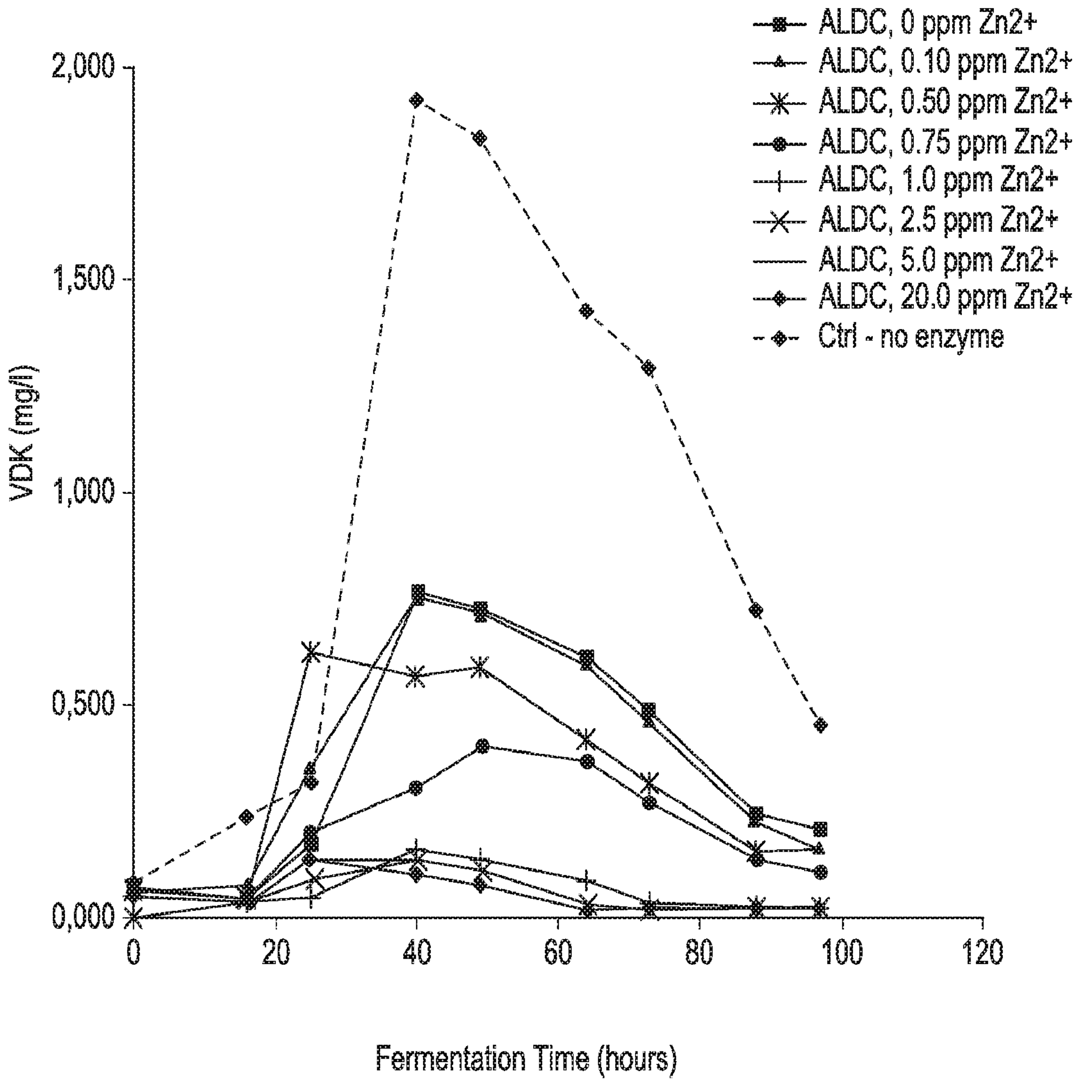
ALDC, 0 ppm Zn2+
ALDC, 0.10 ppm Zn2+
ALDC, 0.50 ppm Zn2+
ALDC, 0.75 ppm Zn2+
ALDC, 1.0 ppm Zn2+
ALDC, 2.5 ppm Zn2+
ALDC, 5.0 ppm Zn2+
ALDC, 20.0 ppm Zn2+
Ctrl - no enzyme
VDK (mg/l)
2,000
1,500
1,000
0,500
0,000
0
20
40
60
80
100
120
Fermentation Time (hours)

*FIG. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DK 149335 B
- US 5874276 A
- US 5246853 A
- US 5475101 A
- WO 9206209 A
- US 5847276 A
- WO 05001036 A
- WO 0214490 A
- EP 238023 A
- US 6022725 A
- US 6268328 B
- EP 244234 A
- EP 215594 A
- WO 9600787 A
- US 62165671 B
- US 62168406 B
- US 62166610 B


### Non-patent literature cited in the description


- **SINGLETON et al.** DICTIONARY OF MICROBIOLOGY AND MOLECULAR BIOLOGY. John Wiley and Sons, 1994
- **HALE ; MARHAM.** THE HARPER COLLINS DICTIONARY OF BIOLOGY. Harper Perennial, 1991
- More Gene Manipulations In Fungi. Academic Press, 1991, 396-428
- **ALEXOPOULOS, C. J.** Introductory Mycology. Wiley, 1962
- **SHEIR-NEIRS et al.** *Appl. Microbiol. Biotechnol.,* 1984, vol. 20, 46-53
- **WARD et al.** *Appl. Microbiol. Biotechnol.,* 1993, vol. 39, 738-743
- **GOEDEGEBUUR et al.** *Curr. Genet.,* 2002, vol. 41, 89-98
- **INNIS et al.** *Science,* 1985, vol. 228, 21-26
- **CAMPBELL et al.** Curr. Genet. 1989, vol. 16, 53-56
- **ANAGNOSTOPOULOS C. ; J. SPIZIZEN.** *J. Bacteriol.,* 1961, vol. 81, 741-746
- **YELTON et al.** *Proc. Natl. Acad. Sci. USA,* 1984, vol. 81, 1470-1474
- **BERKA et al.** Applications of Enzyme Biotechnology. Plenum Press, 1991
- **CAO et al.** *Protein Sci.,* 2000, vol. 9, 991-1001
- **CAMPBELL et al.** *Curr. Genet.,* 1989, vol. 16, 53-56
- **HARKKI et al.** *Enzyme Microb. Technol.,* 1991, vol. 13, 227-233
- **HARKKI et al.** *BioTechnol.,* 1989, vol. 7, 596-603
- The Molecular Biology of Trichoderma and its Application to the Expression of Both Homologous and Heterologous Genes. **NEVALAINEN et al.** Molecular Industrial Mycology. Marcel Dekker Inc, 1992, 129-148
- **BAJAR et al.** *Proc. Natl. Acad. Sci. USA,* 1991, vol. 88, 8202-8212
- **DIDERICHSEN et al.** *J Bacteriol.,* 1990, vol. 172 (8), 4315
- **MANUELSSON et al.** *Nature Protocols,* 2007, vol. 2, 953-971

SEQUENCE LISTING

<110> DuPont Nutrition Biosciences ApS Cramer, Jacob Flyvholm Jensen, Lene Bojsen Kellett-Smith, Anja Hemmingsen Bladt, Tove Lee, Sang-Kye

<120> Acetolactate Decarboxylase

<130> NB40736 PCT

<150> US62/165671
<151> 2015-05-22

<150> US62/168406
<151> 2015-05-29

<150> US62/166610
<151> 2015-05-26

<160> 8

<170> PatentIn version 3.5

<210> 1
<211> 858
<212> DNA
<213> Brevibacillus brevis

<400> 1

```
atgaaaaaaa atatcatcac ttctatcaca tctctggctc tggttgccgg gctgtctttg       60
actgcttttg cagctacaac ggctactgta ccagcaccac ctgccaagca ggaatccaaa      120
cctgcggttg ccgctaatcc ggcaccaaaa aatgtactgt ttcaatactc aacgatcaat      180
gcactcatgc ttggacagtt tgaaggggac ttgactttga aagacctgaa gctgcgaggc      240
gatatggggc ttggtaccat caatgatctc gatggagaga tgattcagat gggtacaaaa      300
ttctaccaga tcgacagcac cggaaaatta tcggagctgc cagaaagtgt gaaaactcca      360
tttgcggtta ctacacattt cgagccgaaa gaaaaaacta cattaaccaa tgtgcaagat      420
tacaatcaat taacaaaaat gcttgaggag aaatttgaaa acaagaacgt cttttatgcc      480
gtaaagctga ccggtacctt taagatggta aaggctagaa cagttccaaa acaaaccaga      540
ccttatccgc agctgactga agtaaccaaa aaacaatccg agtttgaatt taaaaatgtt      600
aagggaaccc tgattggctt ctatacgcca aattatgcag cagccctgaa tgttcccgga      660
ttccatctcc acttcatcac agaggataaa acaagtggcg gacacgtatt aaatctgcaa      720
tttgacaacg cgaatctgga aatttctccg atccatgagt ttgatgtaca attgccgcac      780
acagatgatt ttgcccactc tgatctgaca caagttacta ctagccaagt acaccaagct      840
gagtcagaaa gaaaataa                                                    858
```

<210> 2
<211> 285
<212> PRT
<213> Brevibacillus brevis

<400> 2

```
Met Lys Lys Asn Ile Ile Thr Ser Ile Thr Ser Leu Ala Leu Val Ala
1               5                   10                  15

Gly Leu Ser Leu Thr Ala Phe Ala Ala Thr Thr Ala Thr Val Pro Ala
            20                  25                  30

Pro Pro Ala Lys Gln Glu Ser Lys Pro Ala Val Ala Ala Asn Pro Ala
        35                  40                  45

Pro Lys Asn Val Leu Phe Gln Tyr Ser Thr Ile Asn Ala Leu Met Leu
    50                  55                  60

Gly Gln Phe Glu Gly Asp Leu Thr Leu Lys Asp Leu Lys Leu Arg Gly
65                  70                  75                  80

Asp Met Gly Leu Gly Thr Ile Asn Asp Leu Asp Gly Glu Met Ile Gln
                85                  90                  95

Met Gly Thr Lys Phe Tyr Gln Ile Asp Ser Thr Gly Lys Leu Ser Glu
            100                 105                 110

Leu Pro Glu Ser Val Lys Thr Pro Phe Ala Val Thr Thr His Phe Glu
        115                 120                 125

Pro Lys Glu Lys Thr Thr Leu Thr Asn Val Gln Asp Tyr Asn Gln Leu
    130                 135                 140

Thr Lys Met Leu Glu Glu Lys Phe Glu Asn Lys Asn Val Phe Tyr Ala
145                 150                 155                 160

Val Lys Leu Thr Gly Thr Phe Lys Met Val Lys Ala Arg Thr Val Pro
                165                 170                 175

Lys Gln Thr Arg Pro Tyr Pro Gln Leu Thr Glu Val Thr Lys Lys Gln
            180                 185                 190

Ser Glu Phe Glu Phe Lys Asn Val Lys Gly Thr Leu Ile Gly Phe Tyr
        195                 200                 205

Thr Pro Asn Tyr Ala Ala Ala Leu Asn Val Pro Gly Phe His Leu His
    210                 215                 220
```

```
Phe Ile Thr Glu Asp Lys Thr Ser Gly Gly His Val Leu Asn Leu Gln
225                 230                 235                 240

Phe Asp Asn Ala Asn Leu Glu Ile Ser Pro Ile His Glu Phe Asp Val
                245                 250                 255

Gln Leu Pro His Thr Asp Asp Phe Ala His Ser Asp Leu Thr Gln Val
            260                 265                 270

Thr Thr Ser Gln Val His Gln Ala Glu Ser Glu Arg Lys
        275                 280                 285
```

<210> 3
<211> 261
<212> PRT
<213> Brevibacillus brevis

<400> 3

```
Ala Thr Thr Ala Thr Val Pro Ala Pro Pro Ala Lys Gln Glu Ser Lys
1               5                   10                  15

Pro Ala Val Ala Ala Asn Pro Ala Pro Lys Asn Val Leu Phe Gln Tyr
            20                  25                  30

Ser Thr Ile Asn Ala Leu Met Leu Gly Gln Phe Glu Gly Asp Leu Thr
        35                  40                  45

Leu Lys Asp Leu Lys Leu Arg Gly Asp Met Gly Leu Gly Thr Ile Asn
    50                  55                  60

Asp Leu Asp Gly Glu Met Ile Gln Met Gly Thr Lys Phe Tyr Gln Ile
65                  70                  75                  80

Asp Ser Thr Gly Lys Leu Ser Glu Leu Pro Glu Ser Val Lys Thr Pro
                85                  90                  95

Phe Ala Val Thr Thr His Phe Glu Pro Lys Glu Lys Thr Thr Leu Thr
            100                 105                 110

Asn Val Gln Asp Tyr Asn Gln Leu Thr Lys Met Leu Glu Glu Lys Phe
        115                 120                 125

Glu Asn Lys Asn Val Phe Tyr Ala Val Lys Leu Thr Gly Thr Phe Lys
    130                 135                 140

Met Val Lys Ala Arg Thr Val Pro Lys Gln Thr Arg Pro Tyr Pro Gln
145                 150                 155                 160
```

```
Leu Thr Glu Val Thr Lys Lys Gln Ser Glu Phe Glu Phe Lys Asn Val
                165                 170                 175

Lys Gly Thr Leu Ile Gly Phe Tyr Thr Pro Asn Tyr Ala Ala Ala Leu
            180                 185                 190

Asn Val Pro Gly Phe His Leu His Phe Ile Thr Glu Asp Lys Thr Ser
        195                 200                 205

Gly Gly His Val Leu Asn Leu Gln Phe Asp Asn Ala Asn Leu Glu Ile
    210                 215                 220

Ser Pro Ile His Glu Phe Asp Val Gln Leu Pro His Thr Asp Asp Phe
225                 230                 235                 240

Ala His Ser Asp Leu Thr Gln Val Thr Thr Ser Gln Val His Gln Ala
                245                 250                 255

Glu Ser Glu Arg Lys
            260
```

<210> 4
<211> 783
<212> DNA
<213> Artificial Sequence

<220>
<223> mature sequence of the aldB gene in plasmid RIHI-aldB

<400> 4

```
gctacaacgg ctactgtacc agcaccacct gccaagcagg aatccaaacc tgcggttgcc      60
gctaatccgg caccaaaaaa tgtactgttt caatactcaa cgatcaatgc actcatgctt     120
ggacagtttg aaggggactt gactttgaaa gacctgaagc tgcgaggcga tatggggctt     180
ggtaccatca atgatctcga tggagagatg attcagatgg gtacaaaatt ctaccagatc     240
gacagcaccg gaaaattatc ggagctgcca gaaagtgtga aaactccatt tgcggttact     300
acacatttcg agccgaaaga aaaaactaca ttaaccaatg tgcaagatta caatcaatta     360
acaaaaatgc ttgaggagaa atttgaaaac aagaacgtct tttatgccgt aaagctgacc     420
ggtactttta agatggtaaa ggctagaaca gttccaaaac aaaccagacc ttatccgcag     480
ctgactgaag taaccaaaaa acaatccgag tttgaattta aaaatgttaa gggaaccctg     540
attggcttct atacgccaaa ttatgcagca gccctgaatg ttcccggatt ccatctccac     600
ttcatcacag aggataaaac aagtggcgga cacgtattaa atctgcaatt tgacaacgcg     660
aatctggaaa tttctccgat ccatgagttt gatgttcaat tgccgcacac agatgatttt     720
gcccactctg atctgacaca agttactact agccaagtac accaagctga gtcagaaaga     780

aaa                                                                   783
```

<210> 5
<211> 290
<212> PRT
<213> Artificial Sequence

<220>
<223> aldB precursor protein expressed from plasmid RIHI-aldB

<400> 5

```
Val Arg Ser Lys Lys Leu Trp Ile Ser Leu Leu Phe Ala Leu Thr Leu
1               5                   10                  15

Ile Phe Thr Met Ala Phe Ser Asn Met Ser Ala Gln Ala Ala Thr Thr
            20                  25                  30

Ala Thr Val Pro Ala Pro Pro Ala Lys Gln Glu Ser Lys Pro Ala Val
        35                  40                  45

Ala Ala Asn Pro Ala Pro Lys Asn Val Leu Phe Gln Tyr Ser Thr Ile
    50                  55                  60

Asn Ala Leu Met Leu Gly Gln Phe Glu Gly Asp Leu Thr Leu Lys Asp
65                  70                  75                  80

Leu Lys Leu Arg Gly Asp Met Gly Leu Gly Thr Ile Asn Asp Leu Asp
                85                  90                  95

Gly Glu Met Ile Gln Met Gly Thr Lys Phe Tyr Gln Ile Asp Ser Thr
            100                 105                 110

Gly Lys Leu Ser Glu Leu Pro Glu Ser Val Lys Thr Pro Phe Ala Val
        115                 120                 125

Thr Thr His Phe Glu Pro Lys Glu Lys Thr Thr Leu Thr Asn Val Gln
    130                 135                 140

Asp Tyr Asn Gln Leu Thr Lys Met Leu Glu Glu Lys Phe Glu Asn Lys
145                 150                 155                 160

Asn Val Phe Tyr Ala Val Lys Leu Thr Gly Thr Phe Lys Met Val Lys
                165                 170                 175

Ala Arg Thr Val Pro Lys Gln Thr Arg Pro Tyr Pro Gln Leu Thr Glu
            180                 185                 190

Val Thr Lys Lys Gln Ser Glu Phe Glu Phe Lys Asn Val Lys Gly Thr
        195                 200                 205
```

```
Leu Ile Gly Phe Tyr Thr Pro Asn Tyr Ala Ala Ala Leu Asn Val Pro
    210                 215                 220

Gly Phe His Leu His Phe Ile Thr Glu Asp Lys Thr Ser Gly Gly His
225                 230                 235                 240

Val Leu Asn Leu Gln Phe Asp Asn Ala Asn Leu Glu Ile Ser Pro Ile
                245                 250                 255

His Glu Phe Asp Val Gln Leu Pro His Thr Asp Asp Phe Ala His Ser
            260                 265                 270

Asp Leu Thr Gln Val Thr Thr Ser Gln Val His Gln Ala Glu Ser Glu
        275                 280                 285

Arg Lys
    290
```

<210> 6
<211> 858
<212> DNA
<213> Artificial Sequence

<220>
<223> sequence encoding an acetolactate decarboxylase

<400> 6

```
atgaaaaaaa atatcatcac ttctatcaca tctctggctc tcgttgccgg gctgtctttg      60
actgcttttg cagctacaac ggctactgta ccagcaccac ctgccaagca ggaatccaaa     120
cctgtggttg ccgctaatcc ggcaccaaaa aatgtactgt ttcaatactc aacgatcaat     180
gcactcatgc ttggacagtt tgaaggggac ttgactttga aagacctgaa gctacgaggc     240
gatatggggc ttggtaccat caatgatctc gatggagaga tgattcagat gggtacaaaa     300
ttctaccaga tcgacagcac cggaaaatta tccgagctgc cagaaagtgt gaaaactcca     360
tttgcggtta ctacacattt cgagccgaaa gaaaaaacta cattaaccaa tgtgcaagat     420
tacaatcaat taacaaaaat gcttgaggag aaatttgaaa acaagaacgt cttttatgcc     480
gtaaagctga ccggtacctt taagatggta aaggctagaa cagttccaaa acaaaccaga     540
ccttatccgc agctgactga agtaaccaaa aaacaatccg agtttgaatt taaaaatgtt     600
aagggaaccc tgattggctt ctatacgcca aattatgcag cagccctgaa tgttcccgga     660
ttccatctcc acttcatcac agaggataaa acaagtggcg gacacgtatt aaatctgcaa     720
tttgacaacg cgaatctgga aatttctccg atccatgagt ttgatgtaca attgccgcac     780
acagatgatt ttgcccactc tgatctgaca caagttacta ctagccaagt acaccaagct     840
```

gagtcagaaa gaaaataa 858

<210> 7
<211> 285
<212> PRT
<213> Artificial Sequence

<220>
<223> sequence of acetolactate decarboxylase (ALDC) precursor aldB

<400> 7

```
Met Lys Lys Asn Ile Ile Thr Ser Ile Thr Ser Leu Ala Leu Val Ala
1               5                   10                  15

Gly Leu Ser Leu Thr Ala Phe Ala Ala Thr Thr Ala Thr Val Pro Ala
            20                  25                  30

Pro Pro Ala Lys Gln Glu Ser Lys Pro Val Val Ala Ala Asn Pro Ala
        35                  40                  45

Pro Lys Asn Val Leu Phe Gln Tyr Ser Thr Ile Asn Ala Leu Met Leu
    50                  55                  60

Gly Gln Phe Glu Gly Asp Leu Thr Leu Lys Asp Leu Lys Leu Arg Gly
65                  70                  75                  80

Asp Met Gly Leu Gly Thr Ile Asn Asp Leu Asp Gly Glu Met Ile Gln
                85                  90                  95

Met Gly Thr Lys Phe Tyr Gln Ile Asp Ser Thr Gly Lys Leu Ser Glu
            100                 105                 110

Leu Pro Glu Ser Val Lys Thr Pro Phe Ala Val Thr Thr His Phe Glu
        115                 120                 125

Pro Lys Glu Lys Thr Thr Leu Thr Asn Val Gln Asp Tyr Asn Gln Leu
    130                 135                 140

Thr Lys Met Leu Glu Glu Lys Phe Glu Asn Lys Asn Val Phe Tyr Ala
145                 150                 155                 160

Val Lys Leu Thr Gly Thr Phe Lys Met Val Lys Ala Arg Thr Val Pro
                165                 170                 175

Lys Gln Thr Arg Pro Tyr Pro Gln Leu Thr Glu Val Thr Lys Lys Gln
            180                 185                 190

Ser Glu Phe Glu Phe Lys Asn Val Lys Gly Thr Leu Ile Gly Phe Tyr
        195                 200                 205
```

```
Thr Pro Asn Tyr Ala Ala Ala Leu Asn Val Pro Gly Phe His Leu His
    210                 215                 220

Phe Ile Thr Glu Asp Lys Thr Ser Gly Gly His Val Leu Asn Leu Gln
225                 230                 235                 240

Phe Asp Asn Ala Asn Leu Glu Ile Ser Pro Ile His Glu Phe Asp Val
                245                 250                 255

Gln Leu Pro His Thr Asp Asp Phe Ala His Ser Asp Leu Thr Gln Val
            260                 265                 270

Thr Thr Ser Gln Val His Gln Ala Glu Ser Glu Arg Lys
        275                 280                 285
```

<210> 8
<211> 261
<212> PRT
<213> Artificial Sequence

<220>
<223> predicted amino acid sequence of the mature acetolactate decarboxylase (ALDC) aldB

<400> 8

```
Ala Thr Thr Ala Thr Val Pro Ala Pro Pro Ala Lys Gln Glu Ser Lys
1               5                   10                  15

Pro Val Val Ala Ala Asn Pro Ala Pro Lys Asn Val Leu Phe Gln Tyr
            20                  25                  30

Ser Thr Ile Asn Ala Leu Met Leu Gly Gln Phe Glu Gly Asp Leu Thr
        35                  40                  45

Leu Lys Asp Leu Lys Leu Arg Gly Asp Met Gly Leu Gly Thr Ile Asn
    50                  55                  60

Asp Leu Asp Gly Glu Met Ile Gln Met Gly Thr Lys Phe Tyr Gln Ile
65                  70                  75                  80

Asp Ser Thr Gly Lys Leu Ser Glu Leu Pro Glu Ser Val Lys Thr Pro
                85                  90                  95

Phe Ala Val Thr Thr His Phe Glu Pro Lys Glu Lys Thr Thr Leu Thr
            100                 105                 110

Asn Val Gln Asp Tyr Asn Gln Leu Thr Lys Met Leu Glu Glu Lys Phe
        115                 120                 125
```

```
Glu Asn Lys Asn Val Phe Tyr Ala Val Lys Leu Thr Gly Thr Phe Lys
    130                 135                 140

Met Val Lys Ala Arg Thr Val Pro Lys Gln Thr Arg Pro Tyr Pro Gln
145                 150                 155                 160

Leu Thr Glu Val Thr Lys Lys Gln Ser Glu Phe Glu Phe Lys Asn Val
                165                 170                 175

Lys Gly Thr Leu Ile Gly Phe Tyr Thr Pro Asn Tyr Ala Ala Ala Leu
            180                 185                 190

Asn Val Pro Gly Phe His Leu His Phe Ile Thr Glu Asp Lys Thr Ser
        195                 200                 205

Gly Gly His Val Leu Asn Leu Gln Phe Asp Asn Ala Asn Leu Glu Ile
    210                 215                 220

Ser Pro Ile His Glu Phe Asp Val Gln Leu Pro His Thr Asp Asp Phe
225                 230                 235                 240

Ala His Ser Asp Leu Thr Gln Val Thr Thr Ser Gln Val His Gln Ala
                245                 250                 255

Glu Ser Glu Arg Lys
            260
```